# EUROPEAN PATENT APPLICATION

(11) **EP 2 549 399 A1**
(43) Date of publication of application: **23.01.2013**
(21) Application number: 11178148.0
(22) Date of filing: 19.08.2011
(51) Int. Cl.: G06F 19/24, C12Q 1/68, G06F 19/20

(54) **Assessment of Wnt pathway activity using probabilistic modeling of target gene expression**

(30) Priority: 19.07.2011 US 509137 P
(71) Applicant: Koninklijke Philips Electronics N.V., 5621 BA Eindhoven (NL)
(72) Inventor: The designation of the inventor has not yet been filed
(74) Representative: Jungen, Carmen

(57) **Abstract**

Activity of a cellular signaling pathway in tissue of a medical subject is inferred based at least on expression levels of target genes of the cellular signaling pathway measured in an extracted sample of the tissue of the medical subject. It is determined whether the cellular signaling pathway is operating abnormally in the tissue of the medical subject based on the inferred activity of the cellular signaling pathway. The inferring is suitably performed by a digital processing device using a probabilistic model of the cellular signaling pathway, such as a Bayesian network model. The cellular signaling pathway may be a Wnt pathway for which the target genes are suitably selected from the group: KIAA1199, AXIN2, CD44, RNF43, MYC, TBX3, TDGF1, SOX9, ASCL2, IL8, SP5, ZNRF3, EPHB2, LGR5, EPHB3, KLF6, CCND1, DEFA6, FZD7, NKD1, OAT, FAT1, LEF1, GLUL, REG1B, TCF7L2, COL18A1, BMP7, SLC1A2, ADRA2C, PPARG, DKK1, HNF1A, and LECT2.

## Description

The following relates to bioinformatics, genomic processing arts, proteomic processing arts, and related arts.

Genomic and proteomic analyses have substantial realized and potential promise for clinical application in medical fields such as oncology, where various cancers are known to be associated with specific combinations of gene variations and/or high or low expression levels for specific genes. For example, the Wnt signaling pathway affects regulation of cell multiplication, and is highly regulated. High Wnt pathway activity due to loss of regulation has been correlated with malignant colon tumors. While not being limited to any particular theory of operation, it is believed that regulatory failure of the Wnt pathway in malignant colon cells leads to high Wnt pathway activity that in turn causes multiplication of the malignant colon cells, i.e. spread of colon cancer.

Technologies for acquiring genomic and proteomic data have become readily available in clinical settings. For example, microarrays are routinely employed to assess gene expression levels, protein levels, methylation, and so forth. Automated gene sequencing enables cost-effective identification of genetic variations in DNA and mRNA. Quantitative assessment of mRNA levels during gene sequencing holds promise as yet another clinical tool for assessing gene expression levels.

In spite of (or, perhaps, because of) these advances, clinical application of genomic and proteomic analyses faces a substantial hurdle - data overload. For example, the number of identifiable mutations in a single clinical sample can number in the hundreds of thousands or more. A single microarray can generate gene expression levels for tens of thousands of genes. Processing these large quantities of data to identify clinically useful information is difficult.

One approach is to limit the analysis to a few canonical or standardized tests, such as tests approved by the U.S. Food and Drug Administration (FDA). In such an approach, a specific combination of indicators (e.g., mutations and/or specified high or low gene expression levels) is detected in order to test "positive" for the indicated disease condition (e.g., a particular type of cancer). The canonical test is supported by clinical studies that have shown strong correlation for the disease condition. This approach is useful only in diagnosing diseases for which a canonical test has been developed, and is also rigid as it is only applicable for the canonical conditions.

Another approach is based on identification of functionally related groups of genomic or proteomic indicators. For example, the Wnt pathway comprises a cascade of proteomic reactions. Major components of this chain include (but are not limited to) binding of the Wnt signaling protein to a frizzled surface receptor of the cell which causes activation of proteins of the disheveled family of proteins which in turn impact the level of transcription agents such as β-catenin/TCF4 based protein complexes in the cell nucleus. These transcription agents, in turn, control transcription of target mRNA molecules that in turn are translated into target proteins of the Wnt pathway. Clinical studies have shown some correlations between regulatory proteins of the Wnt pathway and the activity of the Wnt pathway.

However, applying such clinical study results to the diagnosis and clinical evaluation of a specific patient is difficult due to the complexity of the Wnt pathway. As a simple example, measurement of the expression level of a protein that is "upstream" in the Wnt pathway may fail to detect abnormal behavior of a protein that is "downstream" in the Wnt pathway. (It is believed that the Wnt pathway includes numerous feedback mechanisms and the simplified concept of "upstream" and "downstream" may be inapplicable for a substantial portion of the Wnt pathway; more generally, abnormal behavior in one portion of the protein cascade comprising the Wnt pathway may have more or less effect on other portions of the protein cascade, and on the activity of the Wnt pathway as a whole). Still further, in some clinical studies protein expression levels for regulatory proteins of the signaling cascade are assessed by measuring mRNA expression levels of the genes that encode for the regulatory proteins. This is an indirect measurement that may not accurately assess the regulatory protein expression level.

The following provides new and improved apparatuses and methods as disclosed herein.

In accordance with one disclosed aspect, a method comprises: inferring activity of a cellular signaling pathway in tissue of a medical subject based at least on expression levels of target genes of the cellular signaling pathway measured in an extracted sample of the tissue of the medical subject; and determining whether the cellular signaling pathway is operating abnormally in the tissue of the medical subject based on the inferred activity of the cellular signaling pathway in the tissue of the medical subject; wherein the inferring is performed by a digital processing device using a probabilistic model of the cellular signaling pathway. The probabilistic model may be a Bayesian network model. The cellular signaling pathway may be a Wnt pathway.

In accordance with another disclosed aspect, a method comprises: inferring activity of a Wnt pathway in tissue of a medical subject based at least on expression levels of target genes of a set of target genes of the Wnt pathway measured in an extracted sample of the tissue of the medical subject; and determining whether the Wnt pathway is operating abnormally in the tissue of the medical subject based on the inferred activity of the Wnt pathway in the tissue of the medical subject; wherein the inferring is performed by a digital processing device using pre-determined conditional probabilities relating levels of the target genes of the set of target genes of the Wnt pathway and a transcription factor (TF) element controlling transcription of the target genes of the set of target genes of the Wnt pathway. In some such embodiments the set of target genes of the Wnt pathway includes target genes selected from a group consisting of: KIAA1199, AXIN2, CD44, RNF43, MYC, TBX3, TDGF1, SOX9, ASCL2, IL8, SP5, ZNRF3, EPHB2, LGR5, EPHB3, KLF6, CCND1, DEFA6, and FZD7. In some such embodiments the set of target genes of the Wnt pathway further includes target genes selected from a group consisting of: NKD1, OAT, FAT1, LEF1, GLUL, REG1B, TCF7L2, COL18A1, BMP7, SLC1A2, ADRA2C, PPARG, DKK1, HNF1A, and LECT2. In some such embodiments the set of target genes of the Wnt pathway includes target genes selected from Table 1 set forth herein. In some embodiments the inferring is performed using a Bayesian network comprising nodes representing information about the Wnt pathway and conditional probability relationships between connected nodes of the Bayesian network.

In accordance with another disclosed aspect, a digital processor is disclosed which is configured to perform a method as set forth in any one of the two immediately preceding paragraphs. In accordance with another disclosed aspect, a non-transitory storage medium stores instructions that are executable by a digital processing device to perform a method as set forth in any one of the two immediately preceding paragraphs.

One advantage resides in a clinical decision support (CDS) system providing clinical recommendations based on probabilistic analysis of a cellular signaling pathway, for example using a Bayesian network model of a Wnt pathway.

Another advantage resides in improved assessment of cellular signaling pathway activity that is less susceptible to error.

Another advantage resides in providing a CDS system recommending targeted treatment for loss of regulation of a cellular signaling pathway.

Another advantage resides in providing a CDS system that is designed to detect loss of regulation for a particular cellular signaling pathway, such as a Wnt pathway, and is readily adapted to provide recommendations for different types of cancer sourced by that particular cellular signaling pathway.

Further advantages will be apparent to those of ordinary skill in the art upon reading and understanding the following detailed description.
- FIGURE 1: diagrammatically shows a clinical decision support (CDS) system configured to assess one or more cellular signaling pathways as disclosed herein.
- FIGURE 2: diagrammatically shows representation of a Wnt pathway using a Bayesian network model and use of that model in assessing Wnt pathway activity.
- FIGURE 3: diagrammatically shows an illustrative embodiment of the Bayesian network model of FIGURE 2.
- FIGURE 4: diagrammatically shows use of the Bayesian network model of FIGURE 3 for inferring Wnt pathway activity based on inputs comprising measured expression levels of target genes of the Wnt pathway measured in an extracted sample of the tissue of the medical subject.
- FIGURE 5: diagrammatically shows a variant embodiment of the Bayesian network model of FIGURE 3 which includes nodes representing methylation data for the target genes of the Wnt pathway.
- FIGURE 6: diagrammatically shows a variant embodiment of the Bayesian network model of FIGURE 3 which includes nodes representing specific measurements of expression levels of target genes of the Wnt pathway.
- FIGURE 7: diagrammatically shows data sets used for training and testing the Bayesian network model and inference components of the CDS system of FIGURE 1.
- FIGURES 8-12: present some inference results as described herein.

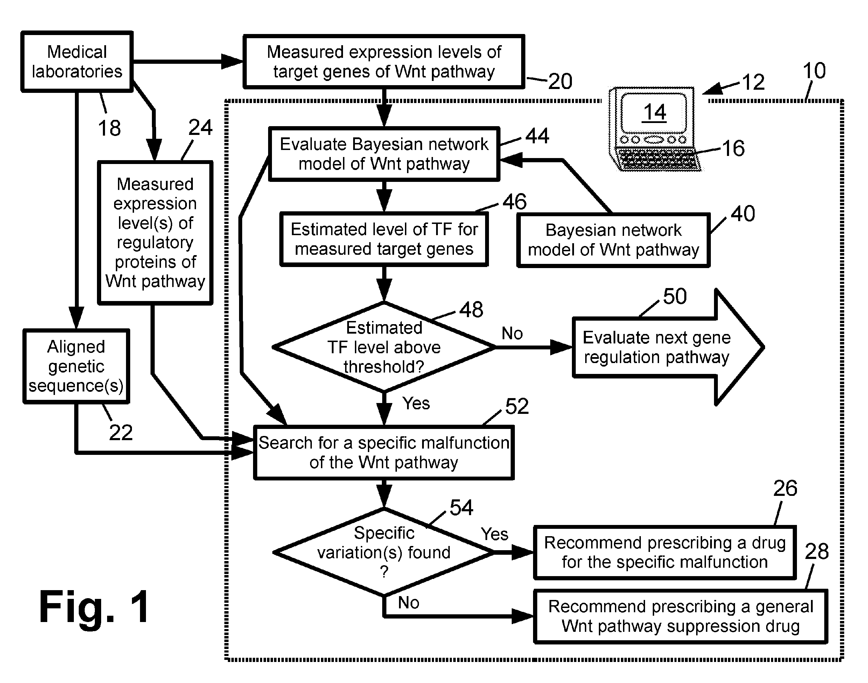

With reference to FIGURE 1, a clinical decision support (CDS) system **10** is implemented as a suitably configured computer **12**. The computer **12** may be configured to operate as the CDS system **10** by executing suitable software, firmware, or other instructions stored on a non-transitory storage medium (not shown) such as a hard drive or other magnetic storage medium, an optical disk or other optical storage medium, a random access memory (RAM), read-only memory (ROM), flash memory, or other electronic storage medium, a network server, or so forth. While the illustrative CDS system **10** is embodied by the illustrative computer **12**, more generally the CDS system may be embodied by a digital processing device or an apparatus comprising a digital processor configured to perform clinical decision support methods as set forth herein. For example, the digital processing device may be a handheld device (e.g., a personal data assistant or smartphone running a CDS application), a notebook computer, a desktop computer, a tablet computer or device, a remote network server, or so forth. The computer **12** or other digital processing device typically includes or is operatively connected with a display device **14** via which information including clinical decision support recommendations are displayed to medical personnel. The computer **12** or other digital processing device typically also includes or is operatively connected with one or more user input devices, such as an illustrative keyboard **16**, or a mouse, trackball, trackpad, touch-sensitive screen (possibly integrated with the display device **14**), or other pointer-based user input device, via which medical personnel can input information such as operational commands for controlling the CDS system **10**, data for use by the CDS system **10**, or so forth.

The CDS system **10** receives as input information pertaining to a medical subject (e.g., a hospital patient, or an outpatient being treated by an oncologist, physician, or other medical personnel, or a person undergoing cancer screening or some other medical diagnosis who is known or suspected to have a certain type of cancer such as colon cancer, breast cancer, or liver cancer, or so forth). The CDS system **10** applies various data analysis algorithms to this input information in order to generate clinical decision support recommendations that are presented to medical personnel via the display device **14** (or via a voice synthesizer or other device providing human-perceptible output). In some embodiments, these algorithms may include applying a clinical guideline to the patient. A clinical guideline is a stored set of standard or "canonical" treatment recommendations, typically constructed based on recommendations of a panel of medical experts and optionally formatted in the form of a clinical "flowchart" to facilitate navigating through the clinical guideline. In various embodiments the data processing algorithms of the CDS **10** may additionally or alternatively include various diagnostic or clinical test algorithms that are performed on input information to extract clinical decision recommendations.

In the illustrative CDS systems disclosed herein (e.g., CDS system **10**), the CDS data analysis algorithms include one or more diagnostic or clinical test algorithms that are performed on input genomic and/or proteomic information acquired by one or more medical laboratories **18**. These laboratories may be variously located "on-site", that is, at the hospital or other location where the medical subject is undergoing medical examination and/or treatment, or "off-site", e.g. a specialized and centralized laboratory that receives (via mail or another delivery service) a sample of tissue of the medical subject that has been extracted from the medical subject (e.g., a sample obtained from a breast lesion, or from a colon of a medical subject known or suspected of having colon cancer, or from a liver of a medical subject known or suspected of having liver cancer, or so forth, via a biopsy procedure or other sample extraction procedure). The tissue of which a sample is extracted may also be metastatic tissue, e.g. (suspected) malignant tissue originating from the colon, breast, liver, or other organ that has spread outside of the colon, breast, liver, or other organ. In some cases, the tissue sample may be circulating tumor cells, that is, tumor cells that have entered the bloodstream and may be extracted as the extracted tissue sample using suitable isolation techniques. The extracted sample is processed by the laboratory to generate genomic or proteomic information. For example, the extracted sample may be processed using a microarray (also variously referred to in the art as a gene chip, DNA chip, biochip, or so forth) or by quantitative polymerase chain reaction (qPCR) processing to measure probative genomic or proteomic information such as expression levels of genes of interest, for example in the form of a level of messenger ribonucleic acid (mRNA) that is transcribed from the gene, or a level of a protein that is translated from the mRNA transcribed from the gene. As another example, the extracted sample may be processed by a gene sequencing laboratory to generate sequences for deoxyribonucleic acid (DNA), or to generate an RNA sequence, or so forth. Other contemplated measurement approaches include immunohistochemistry (IHC), cytology, fluorescence in situ hybridization (FISH), or so forth, performed on a pathology slide. Other information that can be generated by microarray processing, gene sequencing, or other laboratory techniques includes methylation information. Various combinations of such genomic and/or proteomic measurements may also be performed.

In some embodiments, the medical laboratories **18** perform a number of standardized data acquisitions on the extracted sample of the tissue of the medical subject, so as to generate a large quantity of genomic and/or proteomic data. For example, the standardized data acquisition techniques may generate an (optionally aligned) DNA sequence for one or more chromosomes or chromosome portions, or for the entire genome of the tissue. Applying a standard microarray can generate thousands or tens of thousands of data items such as expression levels for a large number of genes, various methylation data, and so forth. This plethora of genomic and/or proteomic data, or selected portions thereof, are input to the CDS system **10** to be processed so as to develop clinically useful information for formulating clinical decision support recommendations.

The disclosed CDS systems and related methods relate to processing of genomic and/or proteomic data to assess activity of various cellular signaling pathways. However, it is to be understood that the disclosed CDS systems (e.g., CDS system **10**) may optionally further include diverse additional capabilities, such as generating clinical decision support recommendations in accordance with stored clinical guidelines based on various patient data such as vital sign monitoring data, patient history data, patient demographic data (e.g., gender, age, or so forth), patient medical imaging data, or so forth. Alternatively, in some embodiments the capabilities of the CDS system **10** may be limited to only performing genomic and/or proteomic data analyses to assess cellular signaling pathways as disclosed herein.

With continuing reference to FIGURE 1, the CDS system **10** infers activity of a cellular signaling pathway in the tissue of the medical subj ect based at least on expression levels of target genes of the cellular signaling pathway measured in the extracted sample, and determines whether the cellular signaling pathway is operating abnormally in the tissue of the medical subject based on this inferred activity. Examples disclosed herein relate to the Wnt pathway as an illustrative cellular signaling pathway. The Wnt pathway is of interest in various areas of oncology because it is believed that loss of regulation of the Wnt pathway can be a cause of (or at least an indicator of) proliferation of certain types of cancers. Without being limited to any particular theory of operation, it is believed that the Wnt pathway regulates cell multiplication, and consequentially a loss of regulation of the Wnt pathway in cancer cells can lead to the Wnt pathway being "always on" thus accelerating the multiplication of cancer cells, which in turn manifests as a spread or metastasis of the cancer.

The CDS system **10** infers activity of the cellular signaling pathway (e.g., the Wnt pathway) based at least on expression levels of target genes of the cellular signaling pathway. This approach recognizes that the expression levels of target genes of the cellular signaling pathway are a direct measure of the activity of the cellular signaling pathway. This is because the cellular signaling pathway directly regulates the transcription of the target genes - hence, the expression levels of mRNA transcribed from the target genes is a direct result of this regulatory activity. In contrast, measurement of mRNA expression levels of genes that encode for regulatory proteins of the cellular signaling pathway, such as an intermediate protein that is part of a protein cascade forming the cellular signaling pathway, is an indirect measure of the regulatory protein expression level and may or may not correlate strongly with the actual regulatory protein expression level (much less with the overall activity of the cellular signaling pathway).

Thus, the CDS system **10** is configured to receive as inputs at least measured expression levels **20** of target genes of the (e.g.) Wnt pathway. This ensures that the CDS system **10** infers the activity of the Wnt pathway based on direct information provided by the measured expression levels of the target genes.

However, although being effective for assessing activity of the overall Wnt pathway, the measured expression levels **20** of target genes of the Wnt pathway are not especially informative as to why the Wnt pathway is operating abnormally (if indeed that is the case). Said another way, the measured expression levels **20** of target genes of the Wnt pathway can indicate that the Wnt pathway is operating abnormally, but do not indicate what portion of the Wnt pathway is malfunctioning (e.g., lacks sufficient regulation) in order to cause the overall Wnt pathway to operate abnormally.

Accordingly, if the CDS system **10** detects abnormal activity of the Wnt pathway, the CDS system **10** then optionally makes use of other information provided by the medical laboratories **18** for the extracted sample, such as aligned genetic sequences **22** and/or measured expression level(s) for one or more regulatory genes of the Wnt pathway **24**, in order to assess what portion of the Wnt pathway is malfunctioning. To maximize efficiency, in some embodiments this optional assessment of why the Wnt pathway is malfunctioning is performed only if the analysis of the measured expression levels **20** of target genes of the Wnt pathway indicates that the Wnt pathway is operating abnormally. On the other hand, if the measured expression levels **20** of target genes of the Wnt pathway indicate that the Wnt pathway is operating normally, then there is no need to probe further. In other embodiments, this assessment is integrated into the probabilistic analysis of the cellular signaling pathway described herein.

In embodiments in which the CDS system **10** assesses what portion of the Wnt pathway is malfunctioning, and is successful in doing so, the additional information enables the CDS system **10** to recommend prescribing a drug targeting for the specific Wnt malfunction (recommendation **26** shown in FIGURE 1). If no specific Wnt malfunction is identified (either because the optional additional assessment is not performed or because that assessment fails to identify any particular portion of the Wnt pathway that is malfunctioning), then the CDS system **10** can provide a default recommendation **28** recommending the prescription of a general Wnt pathway suppression drug (assuming that the abnormal Wnt pathway activity is overly high activity).

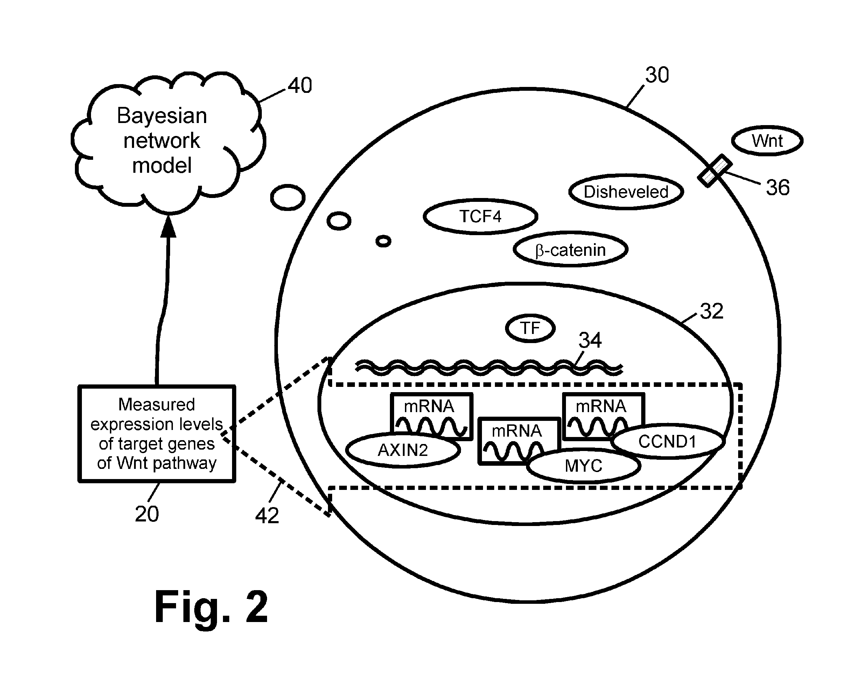

With continuing reference to FIGURE 1 and with further reference to FIGURE 2, operation of the illustrative CDS system **10** is further described. FIGURE 2 diagrammatically illustrates the illustrative Wnt pathway in substantially simplified form. Shown in FIGURE 2 is a cell **30** having a nucleus **32** containing a genome in the form of DNA **34**. In the Wnt signaling pathway, an extracellular Wnt protein binds to a frizzled surface receptor **36** of the cell **30**. This binding triggers a cascade of events including activation of proteins of the disheveled family of proteins which in turn impact the level of transcription agents such as β-catenin/TCF4 based protein complexes in the cell nucleus. In simplified FIGURE 2 only the disheveled, β-catenin, and TCF4 regulatory proteins are diagrammatically shown. The cascade of proteins is believed to include various feedback mechanisms where, for example, a activation of a first protein may trigger activation of a second protein which in turn suppresses activation of the first protein. Such feedback mechanisms can be complex, and may involve varying degrees of interaction between numerous regulatory proteins. It is recognized herein that a comprehensive understanding of the complex protein cascade involved in the Wnt pathway is not necessary to make clinical use of this information. (Indeed, a comprehensive understanding of the Wnt pathway is not currently available in the art, even though it is considered a "well-known" cellular signaling pathway).

A "downstream" result of this protein cascade is generation of a transcription factor (TF) element that forms in the nucleus **32**, or alternatively forms outside the nucleus **32** and then migrates into the nucleus **32**. The TF element may, for example, be a single protein (sometimes called a "transcription factor"), or a protein complex (sometimes called a transcription factor complex), or so forth. In general, a TF element controls transcription of controlled genes of the DNA **34** to generate target messenger ribonucleic acid (mRNA) molecules. This is expression of the target genes at the mRNA level. The mRNA molecules, in turn, are translated by interaction with ribosome molecules to form proteins corresponding to the mRNA molecules and corresponding to the target genes. This is expression of the target genes at the protein level.

With continuing reference to FIGURE 2, the Wnt pathway (or, more generally, the cellular signaling pathway) is represented by a probabilistic model, such as an illustrative Bayesian network **40**. Advantageously, the probabilistic model can incorporate information about the cellular signaling pathway that has been obtained by various clinical studies even if the information is available only in a "soft" form, e.g. percentages of study subjects exhibiting probative characteristics. Such soft information can be incorporated as conditional probabilistic relationships. The probabilistic model also enables information to be incorporated based on partial (rather than comprehensive) knowledge of the cellular signaling pathway, again through the use of conditional probabilities.

For example, a certain gene may be identified as a target gene of the cellular signaling pathway, but the correlation of its expression level with activity of the pathway may be strong or weak, and may vary amongst different clinical study subjects. A weak correlation may suggest that other factors besides the cellular signaling pathway influence expression of that target gene, or may reflect complexities of the cellular signaling pathway (e.g., involvement of multiple transcription factors) which are not presently well understood, or may be attributable to some other, currently unknown, source or perturbation. Nonetheless, the correlation between the target gene and the overall activity of the cellular signaling pathway can be represented by conditional probabilities relating the expression level with the pathway activity.

As disclosed herein, by constructing a probabilistic model (e.g., the illustrative Bayesian model **40**) incorporating conditional probabilistic relationships between expression levels of a number of different target genes and the activity of the cellular signaling pathway, such a model can be used to determine the activity of the cellular signaling pathway with a high degree of accuracy. Moreover, the probabilistic model can be readily updated to incorporate additional knowledge obtained by later clinical studies, by adjusting the conditional probabilities and/or adding new nodes to the model to represent additional information sources. In this way, the probabilistic model can be updated as appropriate to embody the most recent medical knowledge.

The foregoing is illustrated with reference to the illustrative Wnt pathway. In the Wnt pathway, the transcription factor (TF) element is believed to be a protein complex or TF complex (that is, a combination of proteins bound together in a specific structure that performs the function of regulating transcription from the target genes). For other pathways, the TF element may be a single protein. The target genes of the Wnt pathway include thirty-four (34) target genes which are enumerated in Table 1:

**Table 1- Ranked list of target genes of Wnt pathway**

| **Rank** | **Gene** |
|---|---|
| 1 | KIAA1199 |
| 2 | AXIN2 |
| 3 | CD44 |
| 4 | RNF43 |
| 5 | MYC |
| 6 | TBX3 |
| 7 | TDGF1 |
| 8 | SOX9 |
| 9 | ASCL2 |
| 10 | IL8 |
| 11 | SP5 |
| 12 | ZNRF3 |
| 13 | EPHB2 |
| 14 | LGR5 |
| 15 | EPHB3 |
| 16 | KLF6 |
| 17 | CCND1 |
| 18 | DEFA6 |
| 19 | FZD7 |
| 20 | NKD1 |
| 21 | OAT |
| 22 | FAT1 |
| 23 | LEF1 |
| 24 | GLUL |
| 25 | REG1B |
| 26 | TCF7L2 |
| 27 | COL18A1 |
| 28 | BMP7 |
| 29 | SLC1A2 |
| 30 | ADRA2C |
| 31 | PPARG |
| 32 | DKK1 |
| 33 | HNF1A |
| 34 | LECT2 |

The "rank" of Table 1 is an assessment of the importance of the target gene in inferring activity of the Wnt pathway. In general, it is expected that the expression level of target genes of higher rank in Table 1 are likely to be more informative as to the overall activity of the Wnt pathway as compared with target genes of lower rank. However, because of the complexity of the Wnt pathway it is to be understood that more complex interrelationships may exist between the target genes and the Wnt pathway activity - for example, considering expression levels of various combinations of target genes may be more probative than considering (even highly) ranked target genes in isolation. In Wnt pathway modeling reported herein, it has been found that the target genes of Table 1 having ranks 1-19 are of a higher probative nature for predicting the Wnt pathway activity as compared with the lower-ranked target genes of ranks 20-34. Nonetheless, given the relative ease with which acquisition technology such as microarrays can acquire expression levels for large sets of genes, it is contemplated to utilize some or all of the target genes of ranks 1-19, and to optionally additionally use one, two, some, or all of the additional target genes of ranks 20-34, in the Bayesian model **40**.

With continuing reference to FIGURE 2, for illustrative purposes only three of the target genes of the Wnt pathway, namely AXIN2, MYC, and CCND1, are shown transcribed (via the diagrammatically indicated mRNA molecules) and translated into AXIN2 and MYC proteins, respectively. It is to be understood that many, most, or all of the target genes: KIAA1199, AXIN2, CD44, RNF43, MYC, TBX3, TDGF1, SOX9, ASCL2, IL8, SP5, ZNRF3, EPHB2, LGR5, EPHB3, KLF6, CCND1, DEFA6, FZD7, NKD1, OAT, FAT1, LEF1, GLUL, REG1B, TCF7L2, COL18A1, BMP7, SLC1A2, ADRA2C, PPARG, DKK1, HNF1A, and LECT2 are analogously translated into mRNA under regulation by the TF element and then transcribed into proteins. The measurement of the expression levels of the target genes is diagrammatically indicated in FIGURE 2 by a dashed arrow **42**. Either the mRNA expression level or the protein expression level, or both, may be measured to generate the measured expression levels **20** of target genes of the Wnt pathway. The measured expression levels **20** of target genes then serve as inputs to the Bayesian model **40** in order to infer the activity of the Wnt pathway.

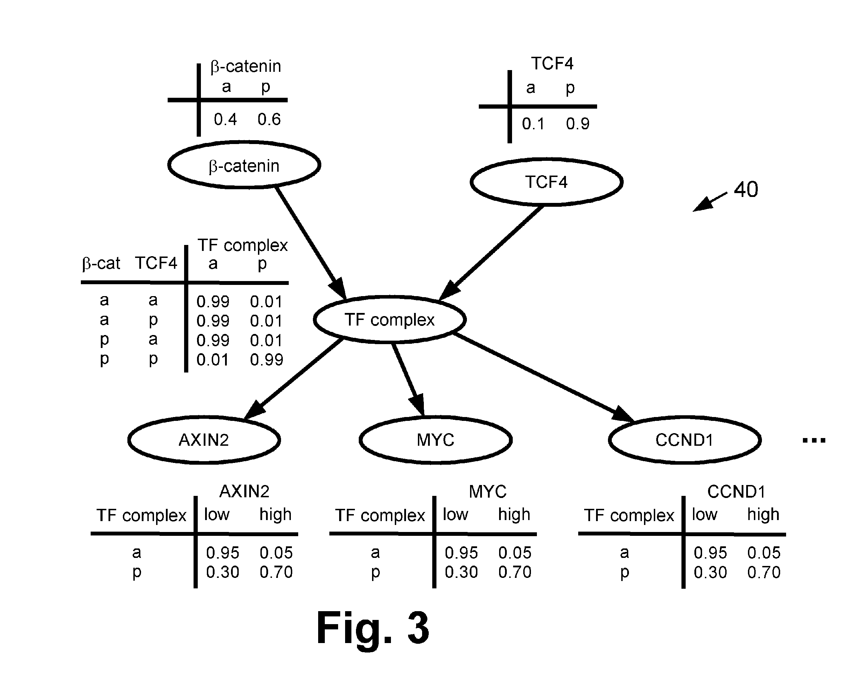

FIGURE 3 shows an illustrative embodiment of the Bayesian network model **40**. In general, a Bayesian network model comprises a directed, acyclic graph comprising nodes connected by edges. Each node represents an information item pertaining to the Wnt pathway (or, more generally, to the cellular signaling pathway). Pathway element nodes each represent a genomic or proteomic element of the cellular signaling pathway. By way of illustrative example, a pathway element node may represent one of: a protein, a protein complex, an mRNA molecule transcribed from a target gene of the cellular signaling pathway, a methylated gene, a phosphorylated protein, a phosphorylated protein complex, or so forth. As discussed later herein, other nodes may be included in the Bayesian network to represent other types of information such as specific measurement datum elements, gene variation occurrences, or so forth. Edges of the Bayesian network indicate conditional probabilistic relationships between the connected nodes.

In the notation used in FIGURE 3, these probabilistic relationships are represented for each pathway element node by a table indicating the conditional probabilistic dependence of the expression level of the represented pathway element (e.g., TF complex, regulatory protein, target gene, or so forth) on the values of the connected nodes. The expression level may be variously represented. In the Bayesian network **40** of FIGURE 1, levels of regulatory proteins (e.g., β-catenin and TCF4 proteins in the illustrative example of FIGURE 3) are represented by the binary quantization of being either "present" (symbolized by "p" in FIGURE 3) or "absent" (symbolized by "a" in FIGURE 3). In the Bayesian network **40** of FIGURE 1, levels of expressed target genes are represented by the binary quantization of "high" or "low", where a level threshold is chosen to differentiate between "high" and "low". Instead of the illustrative binary a/p or high/low values, it is also contemplated to represent the various expression levels as continuous level values or as quantized values having three or more quantization levels (e.g., "high", "normal", or "low" for target genes).

The Bayesian network model of the cellular signaling pathway is a probabilistic model of the pathway. As noted previously, the art generally does not have a comprehensive knowledge of all aspects of any given cellular signaling pathway (even a "well-known" pathway such as the Wnt signaling pathway), and the Bayesian network is typically a substantially simplified model of the pathway. For example, the Bayesian network **40** of FIGURE 3 includes only a single regulatory protein level including the regulatory proteins β-catenin and TCF4, a single TF element (namely the TF protein complex in the case of the Wnt pathway), and the expressed gene levels. This model is substantially simplified versus the known complexity of the Wnt pathway which includes the extracellular Wnt signaling protein, the frizzled surface receptor, and regulatory protein cascade including at least over a dozen different regulatory proteins known in the art to date, some of which interact with each other (which although not shown in FIGURE 3 could be represented in the Bayesian network by edges connecting the interacting regulatory proteins and associated conditional probabilities). The simplest Bayesian network model for representing a cellular signaling pathway would be a two-level model including the TF element and the target genes. Additional "upstream" levels representing regulatory proteins of the pathway are typically added if knowledge of the level of such a protein could be probative for determining the clinical decision support recommendation. For example, the inclusion of the β-catenin in the Bayesian network **40** could be useful if a drug is available that specifically targets the β-catenin protein, rather than the Wnt pathway as a whole.

In the Bayesian network model **40** of FIGURE 3, the various conditional probabilities in the conditional probability tables associated with the various nodes are suitably derived from published clinical studies, and/or from estimates based on current understanding of interactions amongst pathway elements represented by the pathway element nodes. For example, both of the regulatory proteins β-catenin and TCF4 included in the Bayesian network **40** are believed to be component proteins of the TF complex. On first principles, therefore, the TF complex should only be present if both β-catenin and TCF4 are present. Thus, the conditional probability table for the TF complex yields a 99% probability that the TF complex is present if β-catenin and TCF4 are both present, and yields only a 1% probability if either β-catenin or TCF4 is absent. (First principles might suggest that these probabilities should be 100% and 0%, respectively; however, nonzero probability values are advantageous from a computational standpoint, and approximating 100% - 0% by 99% - 1% is unlikely to appreciably affect the inference result).

In both FIGURES 2 and 3, only the three illustrative target genes AXIN2, MYC, and CCND1 are shown for simplicity - however, it is to be understood that any combination of the 34 target genes of Table 1 may be included in the Bayesian model, and moreover it is contemplated to include additional or other target genes of the Wnt pathway that are not listed in Table 1. The Bayesian network **40** assumes a single transcription factor element (namely the illustrative TF complex) regulates transcription of all included target genes of the Wnt pathway. Thus, for N target genes included in the model there are N corresponding edges with each edge connecting one of the target genes with the TF complex. In more complex pathway models (or, possibly, in a more detailed model of the Wnt pathway) there may be two or more TF elements regulating transcription of various target genes of the pathway.

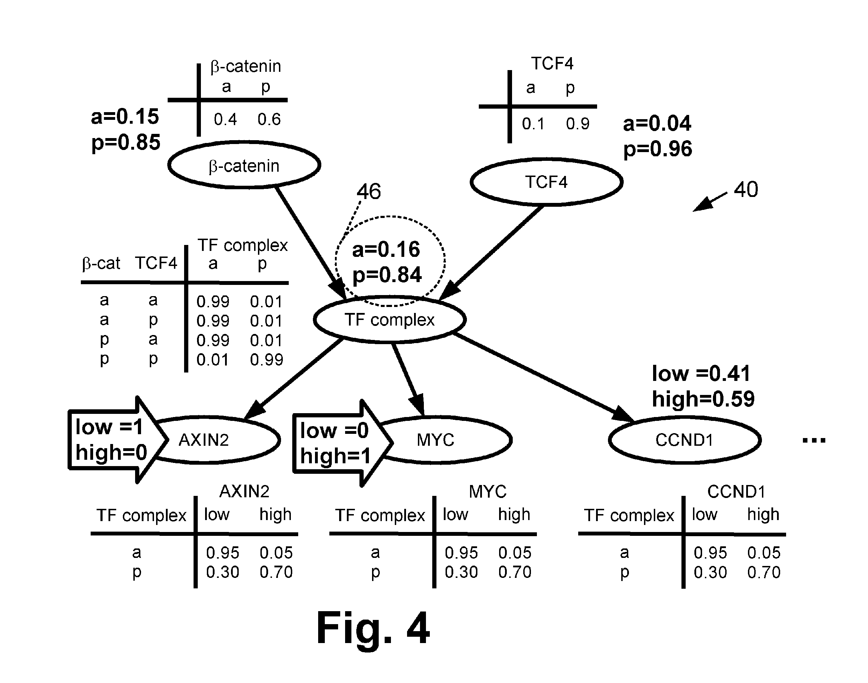

With continuing reference to FIGURE 1 and with further reference to FIGURE 4, the CDS system **10** infers activity of the Wnt pathway based on expression levels of target genes included in the Bayesian network model **40** as follows. FIGURE 4 shows the same Bayesian network **40** as was shown in FIGURE 3, but with inference results superimposed in boldface print. The measured expression levels are known values. However, the measurements may be represented as "soft" evidence, that is, in a probabilistic fashion. By way of illustrative example, if the AXIN2 target gene expression level measurement is below its threshold, but close to it, and if it is known that this measurement has some uncertainty (noise), then the AXIN2 expression level input may be represented as a 0.95 probability of 'low' and a 0.05 probability of 'high' based on suitable estimate of the noise and the difference between the measured mRNA level and the threshold. Such probabilistic soft evidence is readily incorporated into the Bayesian network model **40**.

In the example of FIGURE 4 only the AXIN2 and MYC target gene expression levels are measured: AXIN2 is measured to be at a low level while MYC is measured to be at a high level. These values can be propagated backwards to the TF complex using the conditional probabilities associated with the AXIN2 and MYC nodes (which depend upon the presence or absence of the TF complex). This propagation yields an 84% probability that the TF complex is present, and a 16% probability that the TF complex is absent. This value can optionally be propagated further backwards to the regulatory proteins β-catenin (85% probability of being present, 15% probability of being absent) and TCF4 (96% probability of being present, 4% probability of being absent). The probability values of the TF complex can also be propagated forward to the CCND1 target gene (which is not measured in the instant example of FIGURE 4), yielding a 59% probability that the CCND1 target gene is expressed at a high level and a 41% probability that the CCND target gene is expressed at a low level.

In reviewing illustrative FIGURE 3, it will be noticed that the conditional probabilities are such that a high expression level of the target gene has more impact on the inferred Wnt pathway activity (that is, on the inferred absence/presence probabilities of the TF complex) the than a low expression level. This biasing of the conditional probabilities is expected to be advantageous, because a low expression level for a target gene may have other reasons besides low Wnt pathway activity, such as being caused by methylation of the promotor region of the target gene.

Referring back to FIGURE 1, the inference operation diagrammatically shown in FIGURE 4 corresponds to a Bayesian network evaluation operation **44**. In this evaluation **44**, the presence/absence probabilities **46** associated with the TF complex (see FIGURES 1 and 4) is a suitable metric of the Wnt pathway activity. An active Wnt pathway generates the TF complex, which then drives the expression of the target genes. On the other hand, if the Wnt pathway is not active then the TF complex will be absent and target gene transcription will not occur (or will occur at low target gene expression levels). Thus, in a decision operation **48** (see FIGURE 1) the presence/absence probabilities **46** associated with the TF complex are thresholded (or otherwise analyzed) to determine whether the cellular signaling pathway (here, the illustrative Wnt pathway) is operating abnormally. For oncology applications, a highly active Wnt pathway is considered "abnormal" as it is likely to be driving (or at least associated with) a high rate of multiplication of cancer cells, possibly leading to spread or metastasis of the cancer. Thus, the threshold for the decision operation **48** is selected to detect abnormally high Wnt pathway activity as measured by the presence/absence probabilities **46** associated with the TF complex as inferred by the Bayesian network **40** using the measured expression levels **20** of target genes of the Wnt pathway as inputs.

With continuing reference to FIGURE 1, if the decision operation **48** determines that the Wnt pathway is not abnormally active, then process flows to a "next step" operation **50**. In illustrative FIGURE 1, the "next step" operation **50** is to proceed to analyze another cellular signaling pathway to assess whether it is operating normally or abnormally. While assessment of the Wnt signaling pathway is shown herein as an illustrative example, the disclosed approach of inferring activity of a cellular signaling pathway using a Bayesian network or other probabilistic model of the cellular signaling pathway, and determining whether the cellular signaling pathway is operating abnormally based on the inferred activity, can be readily applied to other cellular signaling pathways. For example, various intracellular signaling pathways, both pathways with receptors in the cell membrane (and usually distinguished or labeled based on the type of receptor), and those pathways with their receptor inside the cell (sometimes called the nuclear receptor family) can be similarly assessed using a Bayesian network or other probabilistic model receiving measured expression levels of target genes of the pathway as inputs to the inference. These intracellular signaling pathways translate the effect of binding a specific ligand, to a functional change in the cell, in terms of a genomic effect (transcription of target genes) resulting in corresponding target mRNA and protein production and longer term functional change, like for example cell division in case of a cancer cell. By way of some further illustrative examples, the disclosed cellular signaling pathway assessment approach is readily adapted to assess intracellular signaling pathways that play a role in early embryonic development. Such cellular signaling pathways are believed to also be relevant for cancer growth (that is, oncogenic applications). Examples of cellular signaling pathways including a membrane receptor include: the Wnt signaling pathway (illustrated herein), Notch, Hedghog, TGFbeta, EGF, VEGF, and TNF-NFkappaB cellular signaling pathways. Examples of cellular signaling pathways including an intracellular receptor include: estrogen, progesterone, androgen, retinoic acid, and vitamin D cellular signaling pathways.

With continuing reference to FIGURE 1, assessing this "next" cellular signaling pathway suitably employs the processing operations **44**, **48**, but with a different Bayesian network designed to model the "next" cellular signaling pathway and with measured expression levels of target genes of the "next" cellular signaling pathway as the inputs to the evaluation operation **44**. Regarding the latter input, a single microarray performing expression level measurements for hundreds, thousands, more different genes and/or proteins can readily supply sufficient expression level data for analyzing a large number of different cellular signaling pathways. Alternatively, rather than evaluating the gene expression pathways sequentially, and moving to the next pathway if the activity estimate for the current one is below the threshold, one may instead evaluate all available pathways in parallel, rank them in order of decreasing value of the estimate **46** of abnormal activity, and select the "most abnormal" pathway or pathways (if any). As yet another approach, a single multi-pathway model can be employed. A multi-pathway model can accommodate overlap between gene expression pathways. For example, it may be the case that a certain node (e.g. target gene) occurs in more than one pathway. For example, a single target gene may be regulated by two transcription elements generated by two different cellular signaling pathways. In such a case, the pathway models that share nodes may be merged into a single multi-pathway model. Doing inference on this multi-pathway model gives concurrent estimates for the activities of all pathways of the multi-pathway model at once.

With returning reference to FIGURE 1, if the decision operation **48** determines that the Wnt pathway is abnormally active (and more specifically operating at an abnormally high level, although it is to be understood that some other cellular signaling pathways may operate at an abnormally low level correlative with an oncological condition), then this information is utilized, either alone or in combination with other information, in formulating a clinical decision support recommendation. In the illustrative CDS system **10** of FIGURE 4, the determination that the Wnt pathway is operating at an abnormally high level in the tissue of the medical subject is followed up by at least one operation **52** which attempts to "narrow down" the specific malfunction of the Wnt pathway. In illustrative FIGURE 1, the follow-up operation **52** is also based on analysis of genomic or proteomic data. For example, the aligned gene sequence **22** may be searched for variants that are known to be associated with specific malfunctions of the Wnt pathway. This sequence search is typically fast, because the total number of genetic variants known to be associated with the Wnt pathway is likely to be relatively low. If a genetic variation associated with malfunction of (by way of illustrative example) the β-catenin regulatory protein is identified, then this may suggest that the abnormal operation of the Wnt pathway is due to this problem with the β-catenin protein. As another illustrative example, the follow-up operation **52** may receive measured expression level(s) **24** for one or more regulatory proteins of the Wnt pathway, and the follow-up operation then uses this information along with the probabilities generated for the regulatory proteins by the Bayesian network evaluation operation **44** in order to assess whether any of these regulatory proteins is at an abnormal expression level. Moreover, if sufficient input (e.g., expression levels for a sufficient number of target genes) is provided to the evaluation operation **44**, it may be possible to determine a likely specific malfunction of the Wnt pathway based on these probabilities without reference to actually measured expression levels of any regulatory proteins of the Wnt pathway.

In some embodiments the follow-up operation **52** may be integrated with the evaluation operation **44**, by extending the inference to include levels of one or more regulatory proteins of the gene expression pathway. When the operation **54** is based on information such as measured regulatory protein expression level(s) **24** and/or genetic variations (e.g., mutations, copy number variations, or so forth) derived from the aligned gene sequence **22**, the integrated approach can provide more accurate inference by basing the inference on a larger set of measured data.

While the illustrative follow-up operation **52** operates on genomic or proteomic data, it is also contemplated that the follow-up operation may operate on other type(s) of data. For example, a laboratory test employing tissue slide staining may be used to narrow down the specific Wnt pathway malfunction, and/or to confirm or validate conclusions initially drawn based on genomic or proteomic data.

In diagrammatic FIGURE 1, the CDS system **10** provides a recommendation **26** (for an identified specific malfunction of the Wnt pathway) or recommendation **28** (for a general malfunction of the Wnt pathway) if the CDS system **10** detects abnormal operation of the Wnt pathway. Optionally, the "next step" **50** is also followed to assess other cellular signaling pathways, or to perform other CDS analyses, and the recommendation **26** or recommendation **28** is combined with recommendations generated by those other CDS steps to formulate a set of recommendations.

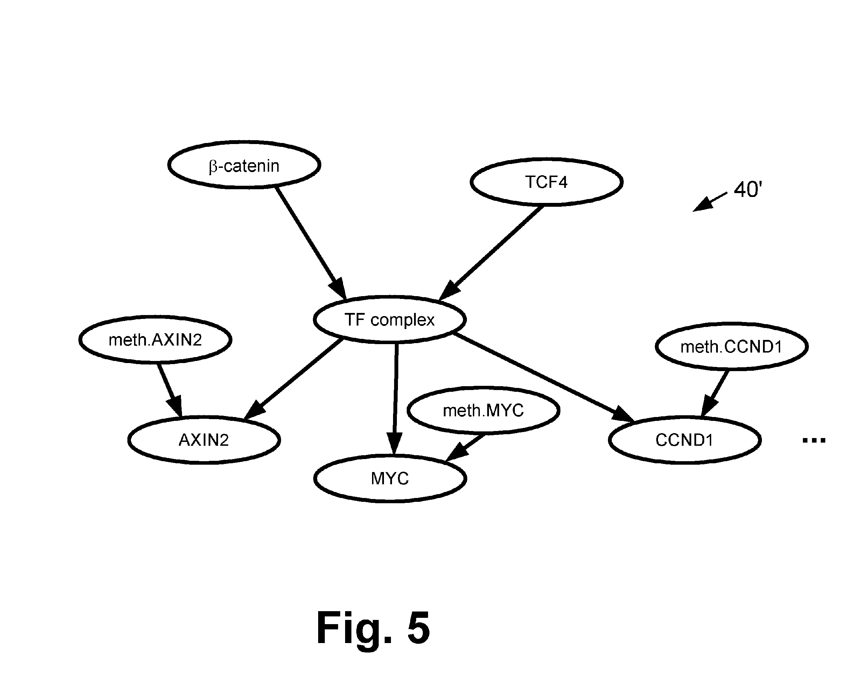

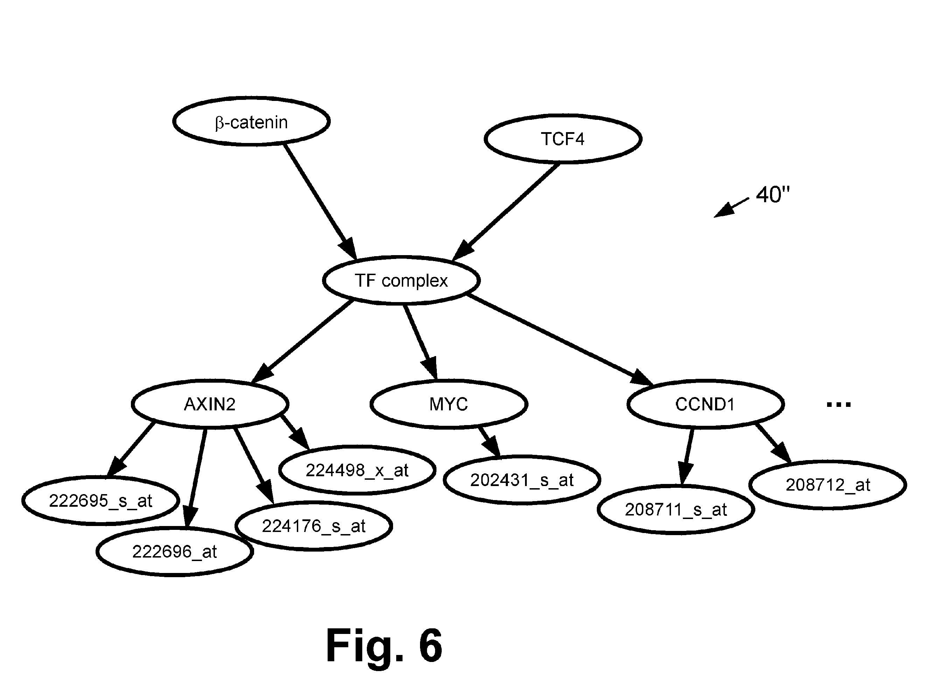

With reference to FIGURES 5 and 6, it is to be appreciated that the Bayesian network **40** may be extended to include other information about the Wnt pathway. FIGURE 5 shows an illustrative variant Bayesian network **40'** that is the same as the Bayesian network **40** shown in FIGURE 3, except that additional nodes are provided to represent methylation data for the target genes AXIN2, MYC, and CCND 1. Thus, if such methylation data are measured they can be inputs for these methylation nodes. FIGURE 6 shows an illustrative variant Bayesian network **40"** that is the same as the Bayesian network **40** shown in FIGURE 3, except that additional nodes are provided to represent the actual expression level measurements for the various target genes of the Wnt pathway. The numbers indicated in the nodes descending from the target gene nodes AXIN2, MYC, and CCND1 identify specific standard measurements performed by the Affymetrix^{®} GeneChip Human Genome U133 Plus 2.0 array preprocessed by the MAS5.0 algorithm (available from Affymetrix, Inc., Santa Clara, California, USA). The conditional probabilities linking each target gene node with its specific measurement nodes implement a desired averaging or other combination of the measurements. Instead of adding the specific measurement nodes as shown in the Bayesian network **40"** of FIGURE 6, the specific measurements can instead be averaged or otherwise combined "off-line" and then input to the Bayesian network model **40** of FIGURE 3.

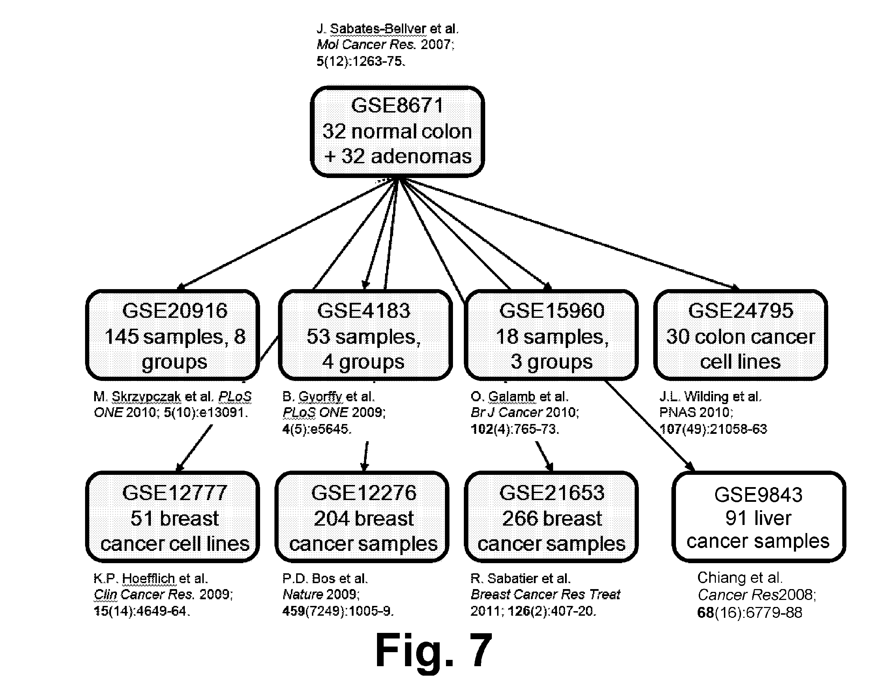

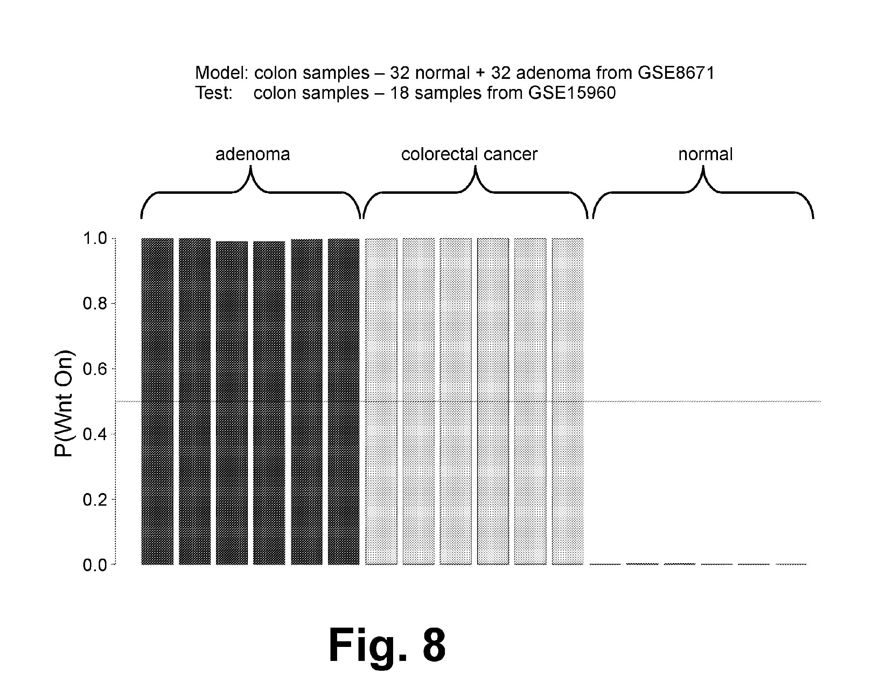

With reference to FIGURE 7, the disclosed CDS system **10** has been applied to various data sets to assess how well the the inference component **40**, **44** operates. The Bayesian network model **40"** was trained using 32 normal colon samples and 32 adenoma samples from data set GSE8671 from the Gene Expression Omnibus (accessible at http://www.ncbi.nlm.nih.gov/geo/, last accessed July 13, 2011). This trained Bayesian network was then tested on various data sets to infer the probability P(Wnt On) that the Wnt pathway is "on", that is, active. Result summaries for some of the test runs are shown in FIGURES 8-12.

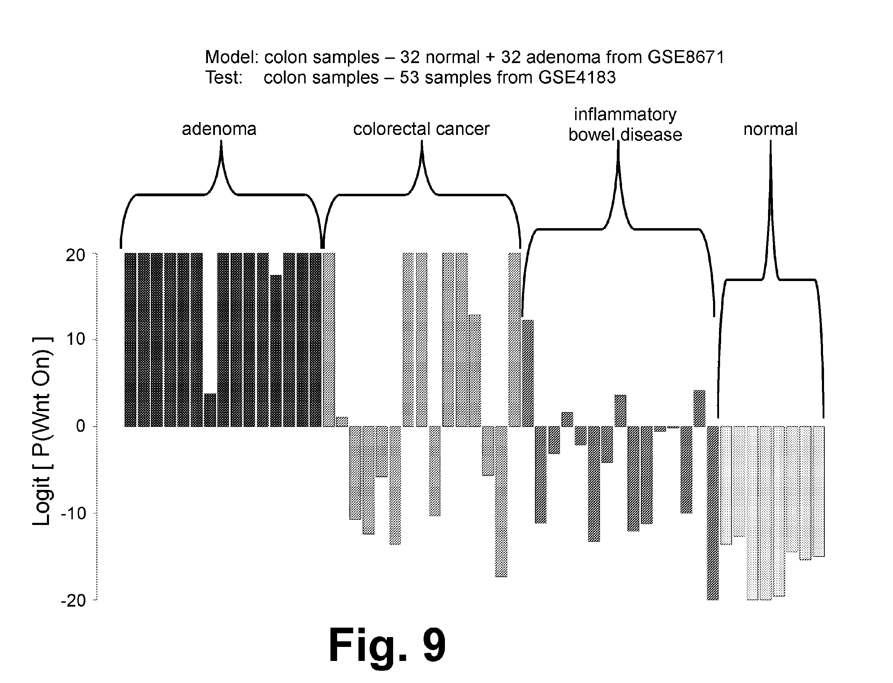

With reference to FIGURES 8 and 9, inference results for two colon sample sets are shown. FIGURE 8 shows results for tests on data set GSE15960, while FIGURE 9 shows results for tests on data set GSE4183.

FIGURE 8 shows strong correlation between P(Wnt On) and the colon sample source. All adenoma samples and all colorectal cancer samples were inferred to have probabilities P(Wnt On) of nearly one; whereas, all normal samples were inferred to have probabilities P(Wnt On) of close to zero. The result for the colorectal samples is consistent with clinical studies that have generally shown strong correlation between colon cancer and high Wnt pathway activity. The result for the adenoma samples was also expected since an adenoma, while benign (that is, not cancerous), still represents a cluster of rapidly multiplying colon cells.

FIGURE 9 plots logit[P(Wnt On)] for another colon sample dataset that further included some samples of inflammatory bowel disease (IBD). Note that the base two logarithm is used in the logit function, i.e. logit(p)=log₂(p)-log₂(1-p). It may also be useful to note that logit(0.5)=0. The Bayesian network model yielded high values of P(Wnt On) for the adenoma samples, and low values for normal samples. For the IBD samples, the Bayesian network model showed low Wnt pathway activity (P(Wnt On)~0) for all but four samples. Again, this is consistent with the IBD samples not undergoing rapid cell multiplication. For the colorectal cancer cell samples the results were mixed, with high Wnt pathway activity being detected in about one-half of these samples.

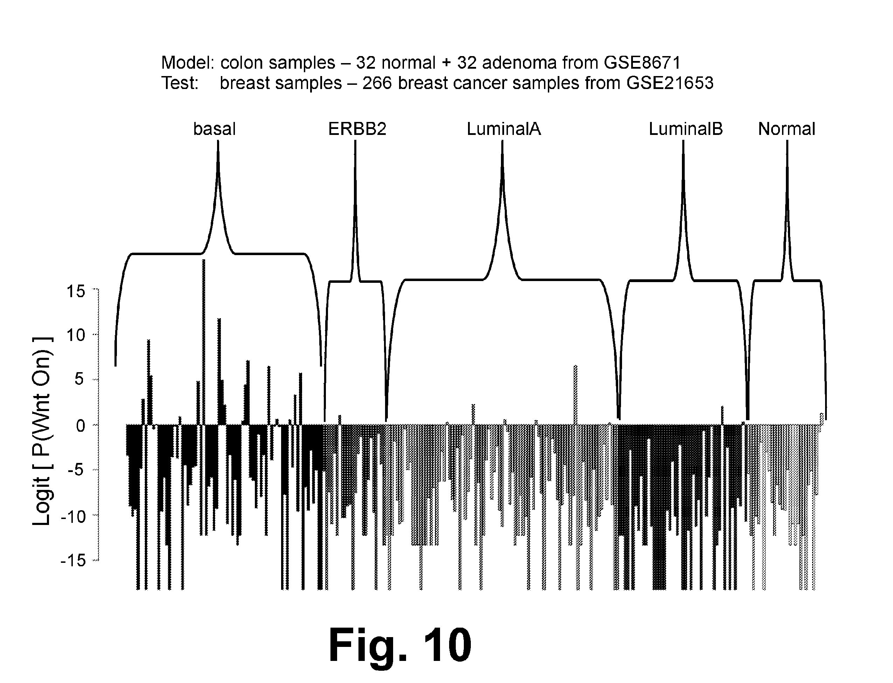

The Bayesian network model used in the experiments reported herein was trained using the colon samples data set GSE8671. However, the Wnt pathway is present (albeit possibly inactive) in all cells. It was therefore considered possible that the Bayesian network might be applicable to infer abnormally high Wnt pathway activity correlative with other types of cancers. The rationale for this is that, although the Bayesian network model was trained using colon samples, it is based on first principles of the operation of the Wnt pathway present (albeit possibly inactive) in all cells. FIGURES 10-12 show some results investigating such "cross-tissue type" inferences.

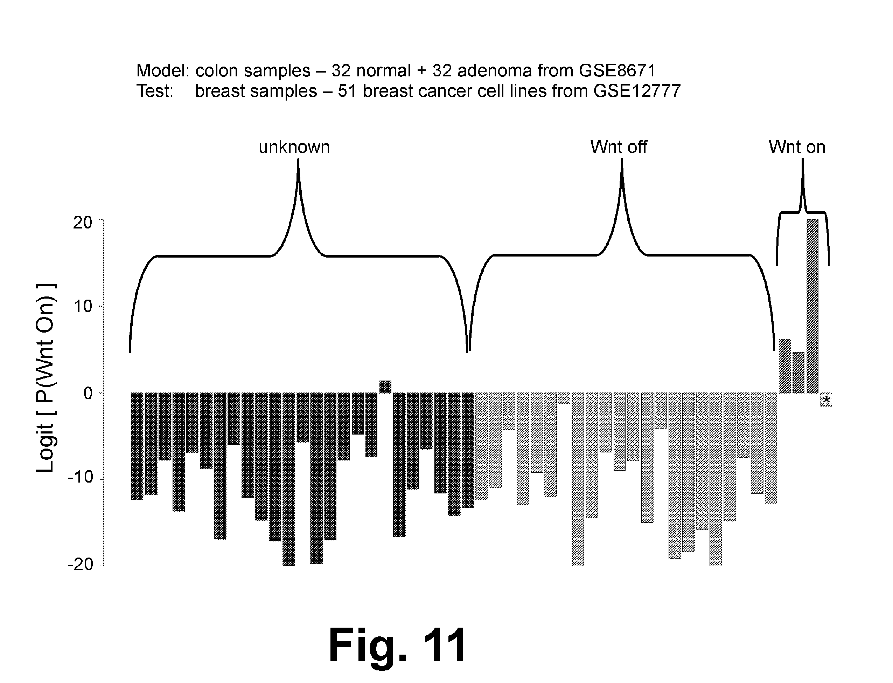

FIGURES 10 and 11 show results for the Bayesian network model trained using colon samples being applied to infer Wnt pathway activity in breast samples. FIGURE 10 shows results for tests on breast data set GSE21653, while FIGURE 11 shows results for tests on breast data set GSE12777.

FIGURE 10 shows results for five different types of breast cancer: basal, ERBB2 (also sometimes known as HER2), Luminal A, Luminal B, and normal-like. As seen in FIGURE 10, the Bayesian network inferred low values for Wnt pathway activity in almost all breast cancer samples of the ERBB2, LuminalA, LuminalB, and normal-like types. The ERBB2, Luminal A, Luminal B, and normal-like types are believed to be associated with cellular signaling pathways other than the Wnt pathway, such as a pathway associated with an estrogen receptor (ER). Thus, these types of breast cancer are not expected to be associated with abnormally high Wnt pathway activity, and the inferences made by the Bayesian network model are consistent with this expectation.

On the other hand, the basal breast cancer type is not known to be strongly associated with any particular cellular signaling pathway (accordingly, the basal type is also sometimes referred to as the "triple-negative" type). One might therefore expect that some, but by no means all or even most, basal breast cancer cases might be associated with abnormally high Wnt pathway activity. The results shown in FIGURE 10 are consistent with this, as about 24% of the basal breast cancer samples show abnormally high Wnt pathway activity. Referring briefly back to FIGURE 1, this result could be the basis for issuing the recommendation **28** to prescribe a Wnt pathway suppression drug in these basal breast cancer cases for which the Bayesian network infers abnormally high Wnt pathway activity; whereas, no such recommendation would be made in the remaining 76% of the basal breast cancer cases for which the Bayesian network did not infer abnormally high Wnt pathway activity.

The results of FIGURE 10 indirectly suggest that the Bayesian network model trained using colon cancer samples is applicable to breast cancer tissue analysis as well. The suggestion is indirect in that the type of breast cancer is used to assess the likelihood that the Wnt pathway is high, and it is this indirect suggestion that substantially correlates with the inferences provided by the Bayesian network.

FIGURE 11 provides more direct evidence. In this case three groups of breast cancer samples are tested: Group I for which the Wnt pathway is *a priori* known to be operating at an abnormally high level; Group II for which the Wnt pathway is *a priori* known to not be operating at an abnormally high level; and Group III for which the Wnt pathway activity is not *a priori* known. As seen in FIGURE 11, the correlation of the inferences provided by the Bayesian network with the *a priori* knowledge is strong for Groups I and II. Indeed, only the rightmost sample of the graph of FIGURE 11 (marked with an asterisk in FIGURE 11) shows an inference at variance with the *a priori* knowledge - in that case a sample *a priori* known to have abnormally high Wnt pathway activity (a member of Group I) is inferred to have a relatively low value for P(Wnt On). Some more recent analysis of that sample has raised questions as to the accuracy of the *a priori* designation of that sample as having abnormally high Wnt pathway activity - nonetheless, even assuming that variant result the correlation for Groups I and II is high.

In the case of Group III shown in FIGURE 11, for which there is no *a priori* knowledge of the Wnt pathway activity, the Bayesian network infers low activity for the Wnt pathway except for one instance for which P(Wnt On)>0.5. In view of the results of FIGURE 10, this is not surprising. Group III is not divided by breast cancer type, and so only a few (and perhaps none) of the samples of Group III are of the basal type. Since FIGURE 10 indicates that the Wnt pathway activity is low for all breast cancer types except the basal type, and further indicates that only about one-in-four samples of basal breast cancer tissue exhibit abnormally high Wnt pathway activity, it is not surprising that Group III shows only one instance of (possibly) abnormally high Wnt pathway activity.

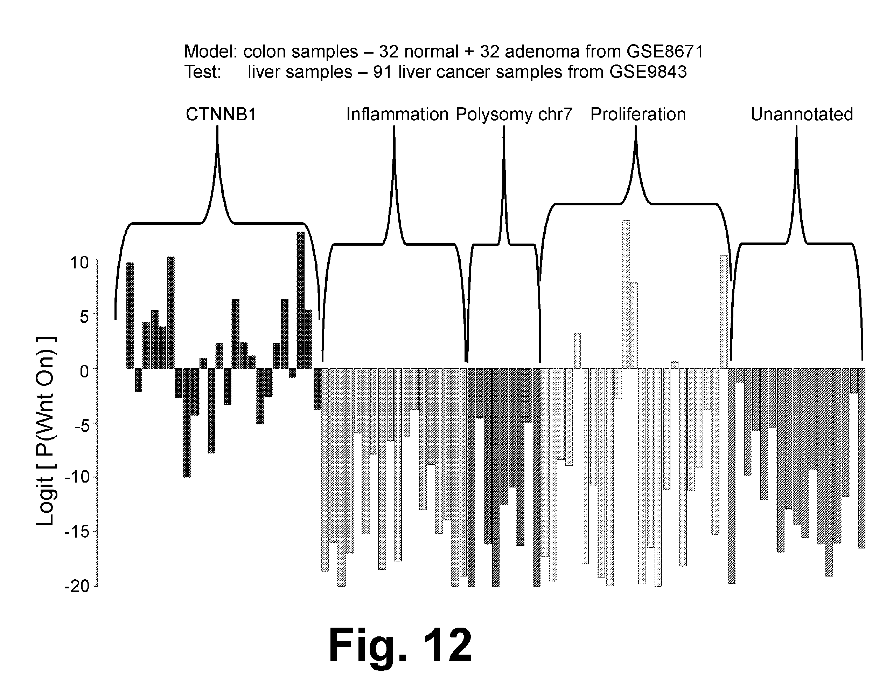

FIGURE 12 shows results for application of the Bayesian network model (which, again, is trained on colon cancer samples) to a different tissue type, in this case liver cancer samples of sample data set GSE9843. Here the samples are grouped by the following *a priori* annotations assigned by the GSE9843 data set: "CTNNB1", "Inflammation", "Polysomy chr7", "Proliferation", and "Unannotated". The samples of the "Inflammation" group are uniformly inferred to not have abnormally high Wnt pathway activity, as expected since the inflammation condition does not entail rapid cell multiplication. Samples labeled "Polysomy chr7" are also uniformly inferred to not have abnormally high Wnt pathway activity. Polysomy of chromosome number 7 means that there are more than two number 7 chromosomes. As there is no reason to expect this polysomy condition to impact the Wnt pathway, it is not unexpected that these samples do not have abnormally high Wnt pathway activity.

About one-in-five of the samples labeled "Proliferation" have P(Wnt On)>0.5. Proliferation suggests a state of rapid cellular multiplication. Such a state may be associated with abnormally high Wnt pathway activity, but may also be associated with numerous other possible causes of cellular multiplication. Accordingly, about one-in-five of these samples having abnormally high Wnt pathway activity is not an unreasonable result.

About one-half of the samples of the "CTNNB 1" group are inferred by the Bayesian network to have abnormally high Wnt pathway activity. The CTNNB 1 gene encodes the β-catenin protein, which is a regulatory protein of the Wnt pathway. Thus, some correlation between the "CTNNB1" group and high Wnt pathway activity is not unexpected.

In summary, the test results for breast cancer and liver cancer sample sets presented in FIGURES 10-12 strongly suggest that the Bayesian network model trained on colon samples is applicable to analysis of samples of other types of tissue. This can enable cellular signaling pathway analysis to be applied "cross-tissue type". Thus, the CDS system **10** is readily applied to assess Wnt pathway activity in a range of tissue types other than the tissue type of the samples used to train the Bayesian network model **40**. In cases where the inference components **40**, **44**, **46**, **48** indicate the tissue under analysis exhibits abnormally high Wnt pathway activity, but no tissue-specific drug is available, a general Wnt pathway suppression drug, or a malfunction-specific drug, may be considered by the physician based on the recommendation **28** or the recommendation **26**, respectively, as provided by the CDS system **10**.

Although the results of FIGURES 10-12 indicate cross-tissue type applicability of the Bayesian network model for the Wnt pathway, it is expected that for clinical applications the Baysian network model may optionally be updated or adapted to maximize its applicability to the specific tissue type under analysis (e.g., breast tissue or liver tissue). Such updating or adaptation could, for example, entail adjusting the conditional probabilities based on clinical studies of the tissue type under analysis. Additionally, nodes might be added or removed to better tune the Bayesian network model to the tissue under analysis. Alternatively, different Bayesian network models may be trained *ab initio* using different training sets for the different tissue types.

In the illustrative examples, approaches are disclosed for assessing the activity of the Wnt pathway in an individual tumor sample. The probability that the Wnt pathway is active is estimated for the one sample under investigation. This estimate is based on the gene expression (transcribed mRNA or translated protein) levels of the target genes of the Wnt pathway, which directly relate to the activity of the Wnt pathway, and in the illustrative examples are not based on genes related to the regulatory proteins involved in the Wnt signaling cascade. The activity assessment is done by means of a probabilistic model, such as the illustrative Bayesian network model. The target gene expression information can come from microarrays, from RNA sequencing experiments, or so forth.

In illustrative examples, the probabilistic model comprises the Bayesian network **40** shown in FIGURE 3. The illustrative Bayesian network **40** includes a node for the TF element (the TF complex for the Wnt pathway model), a node for each of a plurality of target genes (of which only AXIN2, MYC, and CCND1 are shown in FIGURE 3, but further suitable target genes are set forth in Table 1), and arcs or edges running from the TF complex node to each of the target gene nodes. In the illustrative example, each node has a binary value (e.g., absent or present for the TF complex and for any additional regulatory protein nodes, low or high for the target gene nodes, or so forth). However, it is to be understood that a given node can be assigned one of three (or more) levels, or may be assigned a continuous value. Typically, the conditional probabilities of the Bayesian network are selected such that a high expression level of a target gene has more impact on the inferred Wnt pathway activity than a low expression level. This reflects a recognition that a low expression level for a target gene may have other reasons besides low Wnt pathway activity, such as methylation of the gene's promotor region. The expression level of a target gene may be computed based on the measured intensity of corresponding probesets of a microarray, for example by averaging. In some embodiments this computation is integrated into the Bayesian network, by extending the Bayesian network with a node for each probeset that is used and including an arc or edge running to each of these "measurement" nodes from the corresponding target gene node, as described herein with reference to FIGURE 6. The probabilistic model may optionally also incorporate additional genomic information, such as information on mutations, copy number variations, gene expression, methylation information, or so forth, which are related to the signaling cascade of the Wnt pathway to infer the Wnt pathway activity, as described by illustrative reference to FIGURE 5 (for the illustrative case of methylation data).

Moreover, it is to be understood that while examples pertaining to the Wnt pathway are provided as illustrative examples, the approaches for cellular signaling pathway analysis disclosed herein are readily applied to other cellular signaling pathways besides the Wnt pathway, such as to intracellular signaling pathways with receptors in the cell membrane (e.g., the Wnt, Notch, Hedghog, TGFbeta, EGF, VEGF, and TNF-NFkappaB cellular signaling pathways) and intracellular signaling pathways with receptors inside the cell (e.g., estrogen, progesterone, androgen, retinoic acid, and vitamin D cellular signaling pathways). By way of a more specific further example, the non-basal breast cancer samples of the test runs of FIGURE 10 are expected to have substantial correlation with abnormal activity of certain estrogen-related cellular signaling pathways, and so applying the CDS system of FIGURE 1 to such samples with a Bayesian network representing such an estrogen-related cellular signaling pathway and receiving as inputs target genes of that estrogen-related cellular signaling pathway is expected to be a useful analysis tool for assessing abnormal estrogen-related cellular signaling pathway activity that may be of diagnostic or clinical value for non-basal breast cancer cases.

This application has described one or more preferred embodiments. Modifications and alterations may occur to others upon reading and understanding the preceding detailed description. It is intended that the application be construed as including all such modifications and alterations insofar as they come within the scope of the appended claims or the equivalents thereof.

## Claims

1. A method comprising:
inferring activity of a cellular signaling pathway in tissue of a medical subject based at least on expression levels **(20)** of target genes of the cellular signaling pathway measured in an extracted sample of the tissue of the medical subject; and
determining whether the cellular signaling pathway is operating abnormally in the tissue of the medical subject based on the inferred activity of the cellular signaling pathway in the tissue of the medical subject;
wherein the inferring is performed by a digital processing device **(12)** using a probabilistic model **(40, 40', 40")** of the cellular signaling pathway.

2. The method of claim **1**, wherein the inferring comprises:
inferring activity of the cellular signaling pathway in the tissue of the medical subj ect by evaluating at least a portion of a Bayesian network **(40, 40', 40")** representing the cellular signaling pathway for a set of inputs including at least the expression levels **(20)** of target genes of the cellular signaling pathway measured in the extracted sample of the tissue of the medical subject.

3. The method of claim **2**, wherein the inferring comprises:
estimating a level **(46)** in the tissue of the medical subject of a transcription factor (TF) element represented by a TF node of the Bayesian network, the TF element controlling transcription of target genes of the cellular signaling pathway, the estimating being based at least in part on conditional probabilities of the Bayesian network **(40, 40', 40")** relating the TF node and nodes in the Bayesian network representing target genes of the cellular signaling pathway measured in the extracted sample of the tissue of the medical subj ect; and
inferring activity of the cellular signaling pathway based on the estimated level in the tissue sample of the transcription factor.

4. The method of any one of claims **1-3**, wherein the cellular signaling pathway comprises a Wnt pathway.

5. The method of claim **4**, wherein the inferring comprises:
inferring activity of the Wnt pathway in the tissue of the medical subject based at least on expression levels **(20)** of at least three target genes of the Wnt pathway measured in the extracted sample of the tissue of the medical subject selected from a group consisting of: KIAA1199, AXIN2, RNF43, TBX3, TDGF1, SOX9, ASCL2, IL8, SP5, ZNRF3, KLF6, CCND1, DEFA6, and FZD7.

6. The method of claim **5**, wherein the inferring is further based on expression levels **(20)** of at least one target gene of the Wnt pathway measured in the extracted sample of the tissue of the medical subject selected from a group consisting of: NKD1, OAT, FAT1, LEF1, GLUL, REG1B, TCF7L2, COL18A1, BMP7, SLC1A2, ADRA2C, PPARG, DKK1, HNF1A, and LECT2.

7. The method of any one of claims **1-6**, further comprising:
recommending prescribing a drug for the medical subject that corrects for abnormal operation of the cellular signaling pathway;
wherein the recommending is performed only if the cellular signaling pathway is determined to be operating abnormally in the tissue of the medical subject based on the inferred activity of the cellular signaling pathway.

8. A method comprising:
inferring activity of a Wnt pathway in tissue of a medical subject based at least on expression levels **(20)** of target genes of a set of target genes of the Wnt pathway measured in an extracted sample of the tissue of the medical subject; and
determining whether the Wnt pathway is operating abnormally in the tissue of the medical subject based on the inferred activity of the Wnt pathway in the tissue of the medical subject;
wherein the inferring is performed by a digital processing device **(12)** using pre-determined conditional probabilities relating levels of the target genes of the set of target genes of the Wnt pathway and a transcription factor (TF) element controlling transcription of the target genes of the set of target genes of the Wnt pathway.

9. The method of claim **8**, wherein the set of target genes of the Wnt pathway includes at least nine target genes selected from a group consisting of: KIAA1199, AXIN2, CD44, RNF43, MYC, TBX3, TDGF1, SOX9, ASCL2, IL8, SP5, ZNRF3, EPHB2, LGR5, EPHB3, KLF6, CCND1, DEFA6, and FZD7.

10. The method of claim **8**, wherein the set of target genes of the Wnt pathway includes at least one target gene selected from the group consisting of: KIAA1199, AXIN2, RNF43, TBX3, TDGF1, SOX9, ASCL2, IL8, SP5, ZNRF3, KLF6, CCND1, DEFA6, and FZD7.

11. The method of claim **8**, wherein the set of target genes of the Wnt pathway includes at least KIAA1199, AXIN2, RNF43, TBX3, TDGF1, SOX9, ASCL2, IL8, SP5, ZNRF3, KLF6, CCND1, DEFA6, and FZD7.

12. The method of any one of claims **9-11**, wherein the set of target genes of the Wnt pathway further includes at least one target gene selected from a group consisting of:
NKD1, OAT, FAT1, LEF1, GLUL, REG1B, TCF7L2, COL18A1, BMP7, SLC1A2, ADRA2C, PPARG, DKK1, HNF1A, and LECT2.

13. The method of any one of claims **8-12**, wherein the inferring is performed using a Bayesian network **(40, 40', 40")** comprising nodes representing information about the Wnt pathway and conditional probability relationships between connected nodes of the Bayesian network.

14. An apparatus comprising a digital processor **(12)** configured to perform a method as set forth in any one of claims **1-13**.

15. A non-transitory storage medium storing instructions that are executable by a digital processing device **(12)** to perform a method as set forth in any one of claims **1-13**.


SEQUENCE LISTING

<110>  Koninklijke Philips Electronics N.V.

<120>  Assessment of Wnt Pathway Activity Using Probalistic Modelling of
       Target Gene Expression

<130>  2011PF01037

<160>  34

<170>  PatentIn version 3.5

<210>  1
<211>  4086
<212>  DNA
<213>  Homo sapiens


<220>
<221>  KIAA1199
<222>  (1)..(4086)

<400>  1
atgggagctg ctgggaggca ggacttcctc ttcaaggcca tgctgaccat cagctggctc      60

actctgacct gcttccctgg ggccacatcc acagtggctg ctgggtgccc tgaccagagc     120

cctgagttgc aaccctggaa ccctggccat gaccaagacc accatgtgca tatcggccag     180

ggcaagacac tgctgctcac ctcttctgcc acggtctatt ccatccacat ctcagaggga     240

ggcaagctgg tcattaaaga ccacgacgag ccgattgttt tgcgaacccg gcacatcctg     300

attgacaacg gaggagagct gcatgctggg agtgccctct gccctttcca gggcaatttc     360

accatcattt tgtatggaag ggctgatgaa ggtattcagc cggatcctta ctatggtctg     420

aagtacattg gggttggtaa aggaggcgct cttgagttgc atggacagaa aaagctctcc     480

tggacatttc tgaacaagac ccttcaccca ggtggcatgg cagaaggagg ctattttttt     540

gaaaggagct ggggccaccg tggagttatt gttcatgtca tcgaccccaa atcaggcaca     600

gtcatccatt ctgaccggtt tgacacctat agatccaaga aagagagtga acgtctggtc     660

cagtatttga acgcggtgcc cgatggcagg atcctttctg ttgcagtgaa tgatgaaggt     720

tctcgaaatc tggatgacat ggccaggaag gcgatgacca aattgggaag caaacacttc     780

ctgcaccttg gatttagaca cccttggagt tttctaactg tgaaaggaaa tccatcatct     840

tcagtggaag accatattga atatcatgga catcgaggct ctgctgctgc ccgggtattc     900

aaattgttcc agacagagca tggcgaatat ttcaatgttt ctttgtccag tgagtgggtt     960

caagacgtgg agtggacgga gtggttcgat catgataaag tatctcagac taaaggtggg    1020

gagaaaattt cagacctctg gaaagctcac ccaggaaaaa tatgcaatcg tcccattgat    1080

atacaggcca ctacaatgga tggagttaac ctcagcaccg aggttgtcta caaaaaaggc    1140

caggattata ggtttgcttg ctacgaccgg ggcagagcct gccggagcta ccgtgtacgg    1200

ttcctctgtg ggaagcctgt gaggcccaaa ctcacagtca ccattgacac caatgtgaac    1260

agcaccattc tgaacttgga ggataatgta cagtcatgga aacctggaga taccctggtc 1320

attgccagta ctgattactc catgtaccag gcagaagagt tccaggtgct tccctgcaga 1380

tcctgcgccc ccaaccaggt caaagtggca gggaaaccaa tgtacctgca catcggggag 1440

gagatagacg gcgtggacat gcgggcggag gttgggcttc tgagccggaa catcatagtg 1500

atgggggaga tggaggacaa atgctacccc tacagaaacc acatctgcaa tttctttgac 1560

ttcgatacct ttgggggcca catcaagttt gctctgggat ttaaggcagc acacttggag 1620

ggcacggagc tgaagcatat gggacagcag ctggtgggtc agtacccgat tcacttccac 1680

ctggccggtg atgtagacga aaggggaggt tatgacccac ccacatacat cagggacctc 1740

tccatccatc atacattctc tcgctgcgtc acagtccatg gctccaatgg cttgttgatc 1800

aaggacgttg tgggctataa ctctttgggc cactgcttct tcacggaaga tgggccggag 1860

gaacgcaaca cttttgacca ctgtcttggc ctccttgtca agtctggaac cctcctcccc 1920

tcggaccgtg acagcaagat gtgcaagatg atcacagagg actcctaccc ggggtacatc 1980

cccaagccca ggcaagactg caatgctgtg tccaccttct ggatggccaa tcccaacaac 2040

aacctcatca actgtgccgc tgcaggatct gaggaaactg gattttggtt tatttttcac 2100

cacgtaccaa cgggcccctc cgtgggaatg tactccccag gttattcaga gcacattcca 2160

ctgggaaaat tctataacaa ccgagcacat tccaactacc gggctggcat gatcatagac 2220

aacggagtca aaaccaccga ggcctctgcc aaggacaagc ggccgttcct ctcaatcatc 2280

tctgccagat acagccctca ccaggacgcc gacccgctga agccccggga gccggccatc 2340

atcagacact tcattgccta caagaaccag gaccacgggg cctggctgcg cggcggggat 2400

gtgtggctgg acagctgccg gtttgctgac aatggcattg gcctgaccct ggccagtggt 2460

ggaaccttcc cgtatgacga cggctccaag caagagataa agaacagctt gtttgttggc 2520

gagagtggca acgtggggac ggaaatgatg gacaatagga tctggggccc tggcggcttg 2580

gaccatagcg gaaggaccct ccctataggc cagaattttc caattagagg aattcagtta 2640

tatgatggcc ccatcaacat ccaaaactgc actttccgaa agtttgtggc cctggagggc 2700

cggcacacca gcgccctggc cttccgcctg aataatgcct ggcagagctg cccccataac 2760

aacgtgaccg gcattgcctt tgaggacgtt ccgattactt ccagagtgtt cttcggagag 2820

cctgggccct ggttcaacca gctggacatg gatggggata agacatctgt gttccatgac 2880

gtcgacggct ccgtgtccga gtaccctggc tcctacctca cgaagaatga caactggctg 2940

gtccggcacc cagactgcat caatgttccc gactggagag gggccatttg cagtgggtgc 3000

tatgcacaga tgtacattca agcctacaag accagtaacc tgcgaatgaa gatcatcaag 3060

aatgacttcc ccagccaccc tctttacctg gagggggcgc tcaccaggag cacccattac 3120

cagcaatacc aaccggttgt caccctgcag aagggctaca ccatccactg ggaccagacg 3180

gcccccgccg aactcgccat ctggctcatc aacttcaaca agggcgactg gatccgagtg 3240

gggctctgct acccgcgagg caccacattc tccatcctct cggatgttca caatcgcctg 3300

ctgaagcaaa cgtccaagac gggcgtcttc gtgaggacct tgcagatgga caaagtggag 3360

cagagctacc ctggcaggag ccactactac tgggacgagg actcagggct gttgttcctg 3420

aagctgaaag ctcagaacga gagagagaag tttgctttct gctccatgaa aggctgtgag 3480

aggataaaga ttaaagctct gattccaaag aacgcaggcg tcagtgactg cacagccaca 3540

gcttacccca agttcaccga gagggctgtc gtagacgtgc cgatgcccaa gaagctcttt 3600

ggttctcagc tgaaaacaaa ggaccatttc ttggaggtga agatggagag ttccaagcag 3660

cacttcttcc acctctggaa cgacttcgct tacattgaag tggatgggaa gaagtacccc 3720

agttcggagg atggcatcca ggtggtggtg attgacggga accaagggcg cgtggtgagc 3780

cacacgagct tcaggaactc cattctgcaa ggcataccat ggcagctttt caactatgtg 3840

gcgaccatcc ctgacaattc catagtgctt atggcatcaa agggaagata cgtctccaga 3900

ggcccatgga ccagagtgct ggaaaagctt ggggcagaca ggggtctcaa gttgaaagag 3960

caaatggcat tcgttggctt caaaggcagc ttccggccca tctgggtgac actggacact 4020

gaggatcaca aagccaaaat cttccaagtt gtgcccatcc ctgtggtgaa gaagaagaag 4080

ttgtga 4086

<210> 2
<211> 2532
<212> DNA
<213> Homo sapiens

<220>
<221> AXIN2
<222> (1)..(2532)

<400> 2
atgagtagcg ctatgttggt gacttgcctc ccggacccca gcagcagctt ccgtgaggat 60

gccccgcggc ccccagtgcc aggggaagaa ggggagaccc caccgtgtca gccaggggtg 120

ggcaagggcc aggtcaccaa acccatgcct gtctcttcca acaccaggcg gaacgaagat 180

gggttggggg agccggaggg gcgggcatct ccggattccc ctctgacccg gtggaccaag 240

tccttacact ccttattggg cgatcaagac ggtgcttacc tgttccgaac tttcctggag 300

agggagaaat gcgtggatac cttagacttc tggtttgcct gcaatggatt caggcagatg 360

aacctgaagg ataccaaaac tttacgagta gccaaagcga tctacaaaag gtacattgag 420

aacaacagca ttgtctccaa gcagctgaag cctgccacca agacctacat aagagatggc 480

atcaagaagc agcagattga ttccatcatg tttgaccagg cgcagaccga gatccagtcg 540

gtgatggagg aaaatgccta ccagatgttt ttgacttctg atatatacct cgaatatgtg 600

aggagtgggg gagaaaacac agcttacatg agtaatgggg gactcgggag cctaaaggtc 660

gtgtgtggct atctccccac cttgaatgaa gaagaggagt ggacttgtgc cgacttcaag 720

tgcaaacttt cgccaaccgt ggttggcttg tccagcaaaa ctctgagggc cacggcgagt 780

gtgaggtcca cggaaactgt tgacagtgga tacaggtcct tcaagaggag cgatcctgtt 840

aatccttatc acataggttc tggctatgtc tttgcaccag ccaccagcgc caacgacagt 900

gagatatcca gtgatgcgct gacggatgat tccatgtcca tgacggacag cagtgtagat 960

ggaattcctc cttatcgtgt gggcagtaag aaacagctcc agagagaaat gcatcgcagt 1020

gtgaaggcca atggccaagt gtctctacct catttcccga gaacccaccg cctgcccaag 1080

gagatgaccc ccgtggaacc cgccaccttt gcagctgagc tgatctcgag gctggaaaag 1140

ctgaagctgg agttggagag ccgccacagc ctggaggagc gcctgcagca gatccgagag 1200

gatgaagaga gagagggctc cgagctcaca ctcaattcgc gggagggggc gcccacgcag 1260

caccccctct ccctactgcc ctccggcagc tacgaggaag acccgcagac gatactggac 1320

gatcacctgt ccagggtcct caagacccct ggctgccagt ctccaggcgt aggccgctat 1380

agcccccgct cccgctcccc ggaccaccac caccaccacc attcgcagta ccactccctg 1440

ctcccgcccg gtggcaagct gcctcccgcg gccgcctcgc cgggcgcctg ccccctcctc 1500

gggggcaaag gctttgtgac caagcagacg acgaagcatg tccaccacca ctacatccac 1560

caccatgccg tccccaagac caaggaggag atcgaggcgg aggccacgca gcgggtgcac 1620

tgcttctgcc ctgggggcag cgagtattac tgctactcga aatgcaaaag ccactccaag 1680

gctccggaaa ccatgcccag cgagcagttt ggcggcagca gaggcagtac cttgcccaaa 1740

cgcaatggga aaggcacgga gccgggcctg gccctgcccg ccagggaagg agggcccccc 1800

ggcggagctg gggccctgca gcttccccgg gaggaaggag acaggtcgca ggatgtctgg 1860

cagtggatgc tggagagtga gcggcagagc aagcccaagc cccatagtgc ccaaagcaca 1920

aaaaaggcct accccttgga gtctgcccgc tcgtctccag gcgaacgagc cagccggcac 1980

catctgtggg ggggcaacag cgggcacccc cgcaccaccc cccgtgccca cctgttcacc 2040

caggaccctg cgatgcctcc cctgacccca cccaacacgc tggctcagct ggaggaggcc 2100

tgtcgcaggc tagctgaggt gtcgaagccc ccaaagcagc ggtgctgtgt ggccagtcag 2160

cagagggaca ggaatcattc ggccactgtt cagacgggag ccacaccctt ctccaatcca 2220

agcctggctc cagaagatca caaagagcca aagaaactgg caggtgtcca cgcgctccag 2280

gccagtgagt tggttgtcac ttactttttc tgtggggaag aaattccata ccggaggatg 2340

ctgaaggctc agagcttgac cctgggccac tttaaagagc agctcagcaa aaagggaaat 2400

tataggtatt acttcaaaaa agcaagcgat gagtttgcct gtggagcggt gtttgaggag 2460

atctgggagg atgagacggt gctcccgatg tatgaaggcc ggattctggg caaagtggag 2520

cggatcgatt ga 2532

<210>  3
<211>  420
<212>  DNA
<213>  Homo sapiens

<220>
<221>  CD44
<222>  (1)..(420)

<400>  3
atggacaagt tttggtggca cgcagcctgg ggactctgcc tcgtgccgct gagcctggcg      60

cagatcgatt tgaatataac ctgccgcttt gcaggtgtat tccacgtgga gaaaaatggt     120

cgctacagca tctctcggac ggaggccgct gacctctgca aggctttcaa tagcaccttg     180

cccacaatgg cccagatgga gaaagctctg agcatcggat ttgagacctg cagtttgcat     240

tgcagtcaac agtcgaagaa ggtgtgggca gaagaaaaag ctagtgatca acagtggcaa     300

tggagctgtg gaggacagaa agccaagtgg actcaacgga gaggccagca agtctcagga     360

aatggtgcat ttggtgaaca aggagtcgtc agaaactcca gaccagttta tgacagctga     420

<210>  4
<211>  2352
<212>  DNA
<213>  Homo sapiens

<220>
<221>  RNF43
<222>  (1)..(2352)

<400>  4
atgagtggtg gccaccagct gcagctggct gccctctggc cctggctgct gatggctacc      60

ctgcaggcag gctttggacg cacaggactg gtactggcag cagcggtgga gtctgaaaga     120

tcagcagaac agaaagctat tatcagagtg atccccttga aaatggaccc cacaggaaaa     180

ctgaatctca ctttggaagg tgtgtttgct ggtgttgctg aaataactcc agcagaagga     240

aaattaatgc agtcccaccc gctgtacctg tgcaatgcca gtgatgacga caatctggag     300

cctggattca tcagcatcgt caagctggag agtcctcgac gggccccccg cccctgcctg     360

tcactggcta gcaaggctcg gatggcgggt gagcgaggag ccagtgctgt cctctttgac     420

atcactgagg atcgagctgc tgctgagcag ctgcagcagc cgctggggct gacctggcca     480

gtggtgttga tctggggtaa tgacgctgag aagctgatgg agtttgtgta caagaaccaa     540

aaggcccatg tgaggattga gctgaaggag cccccggcct ggccagatta tgatgtgtgg     600

atcctaatga cagtggtggg caccatcttt gtgatcatcc tggcttcggt gctgcgcatc     660

cggtgccgcc cccgccacag caggccggat ccgcttcagc agagaacagc ctgggccatc     720

agccagctgg ccaccaggag gtaccaggcc agctgcaggc aggcccgggg tgagtggcca     780

gactcaggga gcagctgcag ctcagcccct gtgtgtgcca tctgtctgga ggagttctct     840

gaggggcagg agctacgggt catttcctgc ctccatgagt tccatcgtaa ctgtgtggac 900

ccctggttac atcagcatcg gacttgcccc ctctgcatgt tcaacatcac agagggagat 960

tcattttccc agtccctggg accctctcga tcttaccaag aaccaggtcg aagactccac 1020

ctcattcgcc agcatcccgg ccatgcccac taccacctcc ctgctgccta cctgttgggc 1080

ccttcccgga gtgcagtggc tcggccccca cgacctggtc ccttcctgcc atcccaggag 1140

ccaggcatgg gccctcggca tcaccgcttc cccagagctg cacatccccg ggctccagga 1200

gagcagcagc gcctggcagg agcccagcac ccctatgcac aaggctgggg actgagccac 1260

ctccaatcca cctcacagca ccctgctgct tgcccagtgc ccctacgccg ggccaggccc 1320

cctgacagca gtggatctgg agaaagctat tgcacagaac gcagtgggta cctggcagat 1380

gggccagcca gtgactccag ctcagggccc tgtcatggct cttccagtga ctctgtggtc 1440

aactgcacgg acatcagcct acagggggtc catggcagca gttctacttt ctgcagctcc 1500

ctaagcagtg actttgaccc cctagtgtac tgcagcccta aaggggatcc ccagcgagtg 1560

gacatgcagc ctagtgtgac ctctcggcct cgttccttgg actcggtggt gcccacaggg 1620

gaaacccagg tttccagcca tgtccactac caccgccacc ggcaccacca ctacaaaaag 1680

cggttccagt ggcatggcag gaagcctggc ccagaaaccg gagtccccca gtccaggcct 1740

cctattcctc ggacacagcc ccagccagag ccaccttctc ctgatcagca agtcaccaga 1800

tccaactcag cagccccttc ggggcggctc tctaacccac agtgccccag ggccctccct 1860

gagccagccc ctggcccagt tgacgcctcc agcatctgcc ccagtaccag cagtctgttc 1920

aacttgcaaa aatccagcct ctctgcccga cacccacaga ggaaaaggcg gggggtcccc 1980

tccgagccca cccctggctc tcggccccag gatgcaactg tgcacccagc ttgccagatt 2040

tttccccatt acacccccag tgtggcatat ccttggtccc cagaggcaca ccccttgatc 2100

tgtggacctc caggcctgga caagaggctg ctaccagaaa ccccaggccc ctgttactca 2160

aattcacagc cagtgtggtt gtgcctgact cctcgccagc ccctggaacc acatccacct 2220

ggggaggggc cttctgaatg gagttctgac accgcagagg gcaggccatg cccttatccg 2280

cactgccagg tgctgtcggc ccagcctggc tcagaggagg aactcgagga gctgtgtgaa 2340

caggctgtgt ga 2352

<210> 5
<211> 1365
<212> DNA
<213> Homo sapiens

<220>
<221> MYC
<222> (1)..(1365)

<400> 5

ctggattttt ttcgggtagt ggaaaaccag cagcctcccg cgacgatgcc cctcaacgtt 60

agcttcacca acaggaacta tgacctcgac tacgactcgg tgcagccgta tttctactgc 120

gacgaggagg agaacttcta ccagcagcag cagcagagcg agctgcagcc cccggcgccc 180

agcgaggata tctggaagaa attcgagctg ctgcccaccc cgcccctgtc ccctagccgc 240

cgctccgggc tctgctcgcc ctcctacgtt gcggtcacac ccttctccct tcggggagac 300

aacgacggcg gtggcgggag cttctccacg gccgaccagc tggagatggt gaccgagctg 360

ctgggaggag acatggtgaa ccagagtttc atctgcgacc cggacgacga gaccttcatc 420

aaaaacatca tcatccagga ctgtatgtgg agcggcttct cggccgccgc caagctcgtc 480

tcagagaagc tggcctccta ccaggctgcg cgcaaagaca gcggcagccc gaaccccgcc 540

cgcggccaca gcgtctgctc cacctccagc ttgtacctgc aggatctgag cgccgccgcc 600

tcagagtgca tcgacccctc ggtggtcttc ccctaccctc tcaacgacag cagctcgccc 660

aagtcctgcg cctcgcaaga ctccagcgcc ttctctccgt cctcggattc tctgctctcc 720

tcgacggagt cctccccgca gggcagcccc gagcccctgg tgctccatga ggagacaccg 780

cccaccacca gcagcgactc tgaggaggaa caagaagatg aggaagaaat cgatgttgtt 840

tctgtggaaa agaggcaggc tcctggcaaa aggtcagagt ctggatcacc ttctgctgga 900

ggccacagca aacctcctca cagcccactg gtcctcaaga ggtgccacgt ctccacacat 960

cagcacaact acgcagcgcc tccctccact cggaaggact atcctgctgc caagagggtc 1020

aagttggaca gtgtcagagt cctgagacag atcagcaaca accgaaaatg caccagcccc 1080

aggtcctcgg acaccgagga gaatgtcaag aggcgaacac acaacgtctt ggagcgccag 1140

aggaggaacg agctaaaacg gagctttttt gccctgcgtg accagatccc ggagttggaa 1200

aacaatgaaa aggcccccaa ggtagttatc cttaaaaaag ccacagcata catcctgtcc 1260

gtccaagcag aggagcaaaa gctcatttct gaagaggact tgttgcggaa acgacgagaa 1320

cagttgaaac acaaacttga acagctacgg aactcttgtg cgtaa 1365

<210> 6
<211> 2172
<212> DNA
<213> Homo sapiens

<220>
<221> TBX3
<222> (1)..(2172)

<400> 6
atgagcctct ccatgagaga tccggtcatt cctgggacaa gcatggccta ccatccgttc 60

ctacctcacc gggcgccgga cttcgccatg agcgcggtgc tgggtcacca gccgccgttc 120

ttccccgcgc tgacgctgcc tcccaacggc gcggcggcgc tctcgctgcc gggcgccctg 180

gccaagccga tcatggatca attggtgggg gcggccgaga ccggcatccc gttctcctcc 240

ctggggcccc aggcgcatct gaggcctttg aagaccatgg agcccgaaga agaggtggag 300

gacgacccca aggtgcacct ggaggctaaa gaactttggg atcagtttca caagcggggc 360

accgagatgg tcattaccaa gtcgggaagg cgaatgtttc ctccatttaa agtgagatgt 420

tctgggctgg ataaaaaagc caaatacatt ttattgatgg acattatagc tgctgatgac 480

tgtcgttata aatttcacaa ttctcggtgg atggtggctg gtaaggccga ccccgaaatg 540

ccaaagagga tgtacattca cccggacagc cccgctactg gggaacagtg gatgtccaaa 600

gtcgtcactt tccacaaact gaaactcacc aacaacattt cagacaaaca tggatttact 660

atattgaact ccatgcacaa ataccagccc cggttccaca ttgtaagagc caatgacatc 720

ttgaaactcc cttatagtac atttcggaca tacttgttcc ccgaaactga attcatcgct 780

gtgactgcat accagaatga taagataacc cagttaaaaa tagacaacaa cccttttgca 840

aaaggtttcc gggacactgg aaatggccga agagaaaaaa gaaaacagct caccctgcag 900

tccatgaggg tgtttgatga aagacacaaa aaggagaatg ggacctctga tgagtcctcc 960

agtgaacaag cagctttcaa ctgcttcgcc caggcttctt ctccagccgc ctccactgta 1020

gggacatcga acctcaaaga tttatgtccc agcgagggtg agagcgacgc cgaggccgag 1080

agcaaagagg agcatggccc cgaggcctgc gacgcggcca agatctccac caccacgtcg 1140

gaggagccct gccgtgacaa gggcagcccc gcggtcaagg ctcacctttt cgctgctgag 1200

cggccccggg acagcgggcg gctggacaaa gcgtcgcccg actcacgcca tagccccgcc 1260

accatctcgt ccagcactcg cggcctgggc gcggaggagc gcaggagccc ggttcgcgag 1320

ggcacagcgc cggccaaggt ggaagaggcg cgcgcgctcc cgggcaagga ggccttcgcg 1380

ccgctcacgg tgcagacgga cgcggccgcc gcgcacctgg cccagggccc cctgcctggc 1440

ctcggcttcg ccccgggcct ggcgggccaa cagttcttca acgggcaccc gctcttcctg 1500

caccccagcc agtttgccat ggggggcgcc ttctccagca tggcggccgc tggcatgggt 1560

cccctcctgg ccacggtttc tggggcctcc accggtgtct cgggcctgga ttccacggcc 1620

atggcctctg ccgctgcggc gcagggactg tccggggcgt ccgcggccac cctgcccttc 1680

cacctccagc agcacgtcct ggcctctcag ggcctggcca tgtccccttt cggaagcctg 1740

ttcccttacc cctacacgta catggccgca gcggcggccg cctcctctgc ggcagcctcc 1800

agctcggtgc accgccaccc cttcctcaat ctgaacacca tgcgcccgcg gctgcgctac 1860

agcccctact ccatcccggt gccggtcccg gacggcagca gtctgctcac caccgccctg 1920

ccctccatgg cggcggccgc ggggcccctg gacggcaaag tcgccgccct ggccgccagc 1980

ccggcctcgg tggcagtgga ctcgggctct gaactcaaca gccgctcctc cacgctctcc 2040

tccagctcca tgtccttgtc gcccaaactc tgcgcggaga aagaggcggc caccagcgaa 2100

ctgcagagca tccagcggtt ggttagcggc ttggaagcca agccggacag gtcccgcagc 2160

gcgtccccgt ag                                                          2172

<210>  7
<211>  567
<212>  DNA
<213>  Homo sapiens

<220>
<221>  TDGF1
<222>  (1)..(567)

<400>  7
atggactgca ggaagatggc ccgcttctct tacagtgtga tttggatcat ggccatttct        60

aaagtctttg aactgggatt agttgccggg ctgggccatc aggaatttgc tcgtccatct       120

cggggatacc tggccttcag agatgacagc atttggcccc aggaggagcc tgcaattcgg       180

cctcggtctt cccagcgtgt gccgcccatg gggatacagc acagtaagga gctaaacaga       240

acctgctgcc tgaatggggg aacctgcatg ctggggtcct tttgtgcctg ccctccctcc       300

ttctacggac ggaactgtga gcacgatgtg cgcaaagaga actgtgggtc tgtgccccat       360

gacacctggc tgcccaagaa gtgttccctg tgtaaatgct ggcacggtca gctccgctgc       420

tttcctcagg catttctacc cggctgtgat ggccttgtga tggatgagca cctcgtggct       480

tccaggactc cagaactacc accgtctgca cgtactacca cttttatgct agttggcatc       540

tgcctttcta tacaaagcta ctattaa                                           567

<210>  8
<211>  1530
<212>  DNA
<213>  Homo sapiens

<220>
<221>  SOX9
<222>  (1)..(1530)

<400>  8
atgaatctcc tggacccctt catgaagatg accgacgagc aggagaaggg cctgtccggc        60

gcccccagcc ccaccatgtc cgaggactcc gcgggctcgc cctgcccgtc gggctccggc       120

tcggacaccg agaacacgcg gccccaggag aacacgttcc ccaagggcga gcccgatctg       180

aagaaggaga gcgaggagga caagttcccc gtgtgcatcc gcgaggcggt cagccaggtg       240

ctcaaaggct acgactggac gctggtgccc atgccggtgc gcgtcaacgg ctccagcaag       300

aacaagccgc acgtcaagcg gcccatgaac gccttcatgg tgtgggcgca ggcggcgcgc       360

aggaagctcg cggaccagta cccgcacttg cacaacgccg agctcagcaa gacgctgggc       420

aagctctgga gacttctgaa cgagagcgag aagcggccct tcgtggagga ggcggagcgg       480

ctgcgcgtgc agcacaagaa ggaccacccg gattacaagt accagccgcg gcggaggaag       540

tcggtgaaga acgggcaggc ggaggcagag gaggccacgg agcagacgca catctccccc       600

aacgccatct tcaaggcgct gcaggccgac tcgccacact cctcctccgg catgagcgag 660

gtgcactccc ccggcgagca ctcggggcaa tcccagggcc caccgacccc acccaccacc 720

cccaaaaccg acgtgcagcc gggcaaggct gacctgaagc gagaggggcg ccccttgcca 780

gaggggggca gacagccccc tatcgacttc cgcgacgtgg acatcggcga gctgagcagc 840

gacgtcatct ccaacatcga gaccttcgat gtcaacgagt ttgaccagta cctgccgccc 900

aacggccacc cgggggtgcc ggccacgcac ggccaggtca cctacacggg cagctacggc 960

atcagcagca ccgcggccac cccggcgagc gcgggccacg tgtggatgtc caagcagcag 1020

gcgccgccgc cacccccgca gcagccccca caggccccgc cggccccgca ggcgcccccg 1080

cagccgcagg cggcgccccc acagcagccg gcggcacccc cgcagcagcc acaggcgcac 1140

acgctgacca cgctgagcag cgagccgggc cagtcccagc gaacgcacat caagacggag 1200

cagctgagcc ccagccacta cagcgagcag cagcagcact cgccccaaca gatcgcctac 1260

agccccttca acctcccaca ctacagcccc tcctacccgc ccatcacccg ctcacagtac 1320

gactacaccg accaccagaa ctccagctcc tactacagcc acgcggcagg ccagggcacc 1380

ggcctctact ccaccttcac ctacatgaac cccgctcagc gccccatgta cacccccatc 1440

gccgacacct ctggggtccc ttccatcccg cagacccaca gcccccagca ctgggaacaa 1500

cccgtctaca cacagctcac tcgaccttga 1530

<210> 9
<211> 582
<212> DNA
<213> Homo sapiens

<220>
<221> ASCL2
<222> (1)..(582)

<400> 9
atggacggcg gcacactgcc caggtccgcg ccccctgcgc cccccgtccc tgtcggctgc 60

gctgcccggc ggagacccgc gtccccggaa ctgttgcgct gcagccggcg gcggcgaccg 120

gccaccgcag agaccggagg cggcgcagcg gccgtagcgc ggcgcaatga gcgcgagcgc 180

aaccgcgtga agctggtgaa cttgggcttc caggcgctgc ggcagcacgt gccgcacggc 240

ggcgccagca agaagctgag caaggtggag acgctgcgct cagccgtgga gtacatccgc 300

gcgctgcagc gcctgctggc cgagcacgac gccgtgcgca acgcgctggc gggagggctg 360

aggccgcagg ccgtgcggcc gtctgcgccc cgcgggccgc cagggaccac cccggtcgcc 420

gcctcgccct cccgcgcttc ttcgtccccg ggccgcgggg gcagctcgga gcccggctcc 480

ccgcgttccg cctactcgtc ggacgacagc ggctgcgaag gcgcgctgag tcctgcggag 540

cgcgagctac tcgacttctc cagctggtta gggggctact ga 582

<210> 10
<211> 300
<212> DNA
<213> Homo sapiens


<220>
<221> IL8
<222> (1)..(300)

<400> 10
atgacttcca agctggccgt ggctctcttg gcagccttcc tgatttctgc agctctgtgt 60

gaaggtgcag ttttgccaag gagtgctaaa gaacttagat gtcagtgcat aaagacatac 120

tccaaacctt tccaccccaa atttatcaaa gaactgagag tgattgagag tggaccacac 180

tgcgccaaca cagaaattat tgtaaagctt tctgatggaa gagagctctg tctggacccc 240

aaggaaaact gggtgcagag ggttgtggag aagtttttga agagggctga gaattcataa 300


<210> 11
<211> 1197
<212> DNA
<213> Homo sapiens


<220>
<221> SP5
<222> (1)..(1197)

<400> 11
atggccgcgg tggccgtcct ccggaacgac tcgctgcagg cctttctcca ggaccgcacc 60

cccagcgcct ccccggacct gggcaagcac tcgcccctgg cattgctggc cgccacctgt 120

agccgcatcg gccagccggg cgcggcggcg cccccggact tcctgcaggt gccctacgac 180

cccgcgctgg gctcaccctc caggctcttc cacccgtgga ccgccgacat gccggcgcac 240

tcgccaggcg cactgccgcc cccgcatccc agcttggggc tgacgccgca gaagacgcac 300

ctgcagccgt ccttcggggc tgcgcacgag cttcccctta caccccccgc cgacccctcg 360

tacccctacg agttctcgcc ggtcaagatg ctgccctcga gcatggcggc tctgcccgcc 420

agctgcgcgc ccgcctacgt gccctacgcg gcgcaggccg cgctgccgcc aggctactcc 480

aacctgctgc ctccgccgcc gccaccgccc ccgccgccca cctgccgcca gttgtcaccc 540

aacccggccc ccgacgacct cccgtggtgg agcatcccgc aggcgggcgc cgggccgggg 600

gcctccgggg ttccgggaag cggcctctcc ggcgcctgtg ccggggcccc ccacgcgccc 660

cgcttccccg cctctgcggc cgctgctgct gcggccgccg ccgccctaca aagaggcctg 720

gtgttgggcc cgtcggactt tgcgcagtac cagagccaga tcgccgcgct gctgcagacc 780

aaggcccccc tggcggccac ggccaggagg tgccgccgct gccgctgtcc caactgccag 840

gcggcgggcg gcgcccccga ggcggagccg gggaagaaga agcagcacgt gtgccacgtg 900

ccgggctgcg gcaaggtgta cgggaagacg tcgcacctga aggcgcacct gcgctggcac 960

acgggcgagc gacccttcgt gtgcaactgg ctcttctgcg ggaagagctt cacgcgctcg 1020

gacgagctgc agcggcacct gcggactcac acgggcgaga agcgctttgc ctgtcccgag 1080

tgcggcaagc gcttcatgcg cagcgaccac ctcgcgaagc acgtcaagac tcaccagaat 1140

aagaagctca aagtcgctga ggccggggtt aagcgggagg acgcgcggga cctgtga 1197

<210> 12
<211> 2511
<212> DNA
<213> Homo sapiens

<220>
<221> ZNRF3
<222> (1)..(2511)

<400> 12
atgcacccac tgggcctatg taataacaat gacgaagagg acttgtatga atatggctgg 60

gtaggagtgg tgaagctgga acagccagaa ttggacccga aaccatgcct cactgtccta 120

ggcaaggcca agcgagcagt acagcgggga gctactgcag tcatctttga tgtgtctgaa 180

aacccagaag ctattgatca gctgaaccag ggctctgaag acccgctcaa gaggccggtg 240

gtgtatgtga agggtgcaga tgccattaag ctgatgaaca tcgtcaacaa gcagaaagtg 300

gctcgagcaa ggatccagca ccgccctcct cgacaaccca ctgaatactt tgacatgggg 360

attttcctgg ctttcttcgt cgtggtctcc ttggtctgcc tcatcctcct tgtcaaaatc 420

aagctgaagc agcgacgcag tcagaattcc atgaacaggc tggctgtgca ggctctagag 480

aagatggaaa ccagaaagtt caactccaag agcaaggggc gccgggaggg gagctgtggg 540

gccctggaca cactcagcag cagctccacg tccgactgtg ccatctgtct ggagaagtac 600

attgatggag aggagctgcg ggtcatcccc tgtactcacc ggtttcacag gaagtgcgtg 660

gacccctggc tgctgcagca ccacacctgc ccccactgtc ggcacaacat catagaacaa 720

aagggaaacc caagcgcggt gtgtgtggag accagcaacc tctcacgtgg tcggcagcag 780

agggtgaccc tgccggtgca ttaccccggc cgcgtgcaca ggaccaacgc catcccagcc 840

taccctacga ggacaagcat ggactcccac ggcaaccccg tcaccttgct gaccatggac 900

cggcacgggg agcagagcct ctattccccg cagacccccg cctacatccg cagctaccca 960

cccctccacc tggaccacag cctggccgct caccgctgcg gcctggagca ccgggcctac 1020

tccccagccc accccttccg caggcccaag ttgagtggcc gcagcttctc caaggcagct 1080

tgcttctccc agtatgagac catgtaccag cactactact tccagggcct cagctacccg 1140

gagcaggagg ggcagtcccc acctagcctc gcaccccggg gcccggcccg tgcctttcct 1200

ccgagcggca gtggcagcct gctcttcccc accgtggtgc acgtggcccc gccctcccac 1260

ctggagagcg gcagcacgtc cagcttcagc tgctatcacg gccaccgctc ggtgtgcagt 1320

ggctacctgg ccgactgccc aggcagcgac agcagcagca gcagcagctc cggccagtgc 1380

cactgttcct ccagtgactc tgtggtagac tgcactgagg tcagcaacca gggcgtgtac 1440

gggagctgct ccaccttccg cagctccctc agcagcgact atgacccctt catctaccgc 1500

agccggagcc cctgtcgtgc cagtgaggcg gggggctcgg gcagctcggg ccggggacct 1560

gccctgtgct tcgagggctc cccgcctccc gaggagctcc cggcggtgca cagtcatggt 1620

gctgggcggg gcgagccttg gccgggccct gcctctccct cgggggatca ggtgtccacc 1680

tgcagcctgg agatgaacta cagcagcaac tcctccctgg agcacagggg gcccaatagc 1740

tctacctcag aagtggggct cgaggcttct cctggggccg cccctgacct caggaggacc 1800

tggaaggggg gccacgagtt gccgtcgtgt gcctgctgct gcgagcccca gccctcccca 1860

gccgggccta gcgccggagc agctggcagc agcaccttgt tcctggggcc ccacctctac 1920

gagggctctg gcccggcggg tggggagccc cagtcaggaa gctcccaggg cttgtacggc 1980

cttcaccccg accatttgcc caggacagat ggggtgaaat acgagggtct gccctgctgc 2040

ttctatgaag agaagcaggt ggcccgcggg ggcggagggg gcagcggctg ctacactgag 2100

gactactcgg tgagtgtgca gtacacgctc accgaggaac caccgcccgg ctgctacccc 2160

ggggcccggg acctgagcca gcgcatcccc atcattccag aggatgtgga ctgtgatctg 2220

ggcctgccct cggactgcca agggacccac agcctcggct cctggggtgg gacgcgaggc 2280

ccggataccc cacggcccca caggggcctg ggagcaaccc gggaagagga gcgggctctg 2340

tgctgccagg ctagggccct actgcggcct ggctgccctc cggaggaggc gggtgctgtc 2400

agggccaact tccctagtgc cctccaggac actcaggagt ccagcaccac tgccactgag 2460

gctgcaggac cgagatctca ctcagcagac agcagcagcc cgggagcctg a 2511

<210> 13
<211> 2961
<212> DNA
<213> Homo sapiens

<220>
<221> EPHB2
<222> (1)..(2961)

<400> 13
atggctctgc ggaggctggg ggccgcgctg ctgctgctgc cgctgctcgc cgccgtggaa 60

gaaacgctaa tggactccac tacagcgact gctgagctgg gctggatggt gcatcctcca 120

tcagggtggg aagaggtgag tggctacgat gagaacatga acacgatccg cacgtaccag 180

gtgtgcaacg tgtttgagtc aagccagaac aactggctac ggaccaagtt tatccggcgc 240

cgtggcgccc accgcatcca cgtggagatg aagttttcgg tgcgtgactg cagcagcatc 300

cccagcgtgc ctggctcctg caaggagacc ttcaacctct attactatga ggctgacttt 360

gactcggcca ccaagacctt ccccaactgg atggagaatc catgggtgaa ggtggatacc 420

attgcagccg acgagagctt ctcccaggtg gacctgggtg gccgcgtcat gaaaatcaac 480
accgaggtgc ggagcttcgg acctgtgtcc cgcagcggct tctacctggc cttccaggac 540
tatggcggct gcatgtccct catcgccgtg cgtgtcttct accgcaagtg cccccgcatc 600
atccagaatg gcgccatctt ccaggaaacc ctgtcggggg ctgagagcac atcgctggtg 660
gctgcccggg gcagctgcat cgccaatgcg gaagaggtgg atgtacccat caagctctac 720
tgtaacgggg acggcgagtg gctggtgccc atcgggcgct gcatgtgcaa agcaggcttc 780
gaggccgttg agaatggcac cgtctgccga ggttgtccat ctgggacttt caaggccaac 840
caaggggatg aggcctgtac ccactgtccc atcaacagcc ggaccacttc tgaaggggcc 900
accaactgtg tctgccgcaa tggctactac agagcagacc tggaccccct ggacatgccc 960
tgcacaacca tcccctccgc gccccaggct gtgatttcca gtgtcaatga gacctccctc 1020
atgctggagt ggacccctcc ccgcgactcc ggaggccgag aggacctcgt ctacaacatc 1080
atctgcaaga gctgtggctc gggccggggt gcctgcaccc gctgcgggga caatgtacag 1140
tacgcaccac gccagctagg cctgaccgag ccacgcattt acatcagtga cctgctggcc 1200
cacacccagt acaccttcga gatccaggct gtgaacggcg ttactgacca gagccccttc 1260
tcgcctcagt tcgcctctgt gaacatcacc accaaccagg cagctccatc ggcagtgtcc 1320
atcatgcatc aggtgagccg caccgtggac agcattaccc tgtcgtggtc ccagccggac 1380
cagcccaatg gcgtgatcct ggactatgag ctgcagtact atgagaagga gctcagtgag 1440
tacaacgcca cagccataaa aagccccacc aacacggtca ccgtgcaggg cctcaaagcc 1500
ggcgccatct atgtcttcca ggtgcgggca cgcaccgtgg caggttacgg gcgctacagc 1560
ggcaagatgt acttccagac catgacagaa gccgagtacc agacaagcat ccaggagaag 1620
ttgccactca tcatcggctc ctcggccgct ggcctggtct tcctcattgc tgtggttgtc 1680
atcgccatcg tgtgtaacag acgggggttt gagcgtgctg actcggagta cacggacaag 1740
ctgcaacact acaccagtgg ccacatgacc ccaggcatga agatctacat cgatcctttc 1800
acctacgagg accccaacga ggcagtgcgg gagtttgcca aggaaattga catctcctgt 1860
gtcaaaattg agcaggtgat cggagcaggg gagtttggcg aggtctgcag tggccacctg 1920
aagctgccag gcaagagaga gatctttgtg gccatcaaga cgctcaagtc gggctacacg 1980
gagaagcagc gccgggactt cctgagcgaa gcctccatca tgggccagtt cgaccatccc 2040
aacgtcatcc acctggaggg tgtcgtgacc aagagcacac ctgtgatgat catcaccgag 2100
ttcatggaga atggctccct ggactccttt ctccggcaaa acgatgggca gttcacagtc 2160
atccagctgg tgggcatgct tcggggcatc gcagctggca tgaagtacct ggcagacatg 2220
aactatgttc accgtgacct ggctgcccgc aacatcctcg tcaacagcaa cctggtctgc 2280
aaggtgtcgg actttgggct ctcacgcttt ctagaggacg atacctcaga ccccacctac 2340

accagtgccc tgggcggaaa gatccccatc cgctggacag ccccggaagc catccagtac 2400

cggaagttca cctcggccag tgatgtgtgg agctacggca ttgtcatgtg ggaggtgatg 2460

tcctatgggg agcggcccta ctgggacatg accaaccagg atgtaatcaa tgccattgag 2520

caggactatc ggctgccacc gcccatggac tgcccgagcg ccctgcacca actcatgctg 2580

gactgttggc agaaggaccg caaccaccgg cccaagttcg gccaaattgt caacacgcta 2640

gacaagatga tccgcaatcc caacagcctc aaagccatgg cgcccctctc ctctggcatc 2700

aacctgccgc tgctggaccg cacgatcccc gactacacca gctttaacac ggtggacgag 2760

tggctggagg ccatcaagat ggggcagtac aaggagagct tcgccaatgc cggcttcacc 2820

tcctttgacg tcgtgtctca gatgatgatg gaggacattc tccgggttgg ggtcactttg 2880

gctggccacc agaaaaaaat cctgaacagt atccaggtga tgcgggcgca gatgaaccag 2940

attcagtctg tggaggtttg a 2961

<210> 14
<211> 2724
<212> DNA
<213> Homo sapiens

<220>
<221> LGR5
<222> (1)..(2724)

<400> 14
atggacacct cccggctcgg tgtgctcctg tccttgcctg tgctgctgca gctggcgacc 60

gggggcagct ctcccaggtc tggtgtgttg ctgaggggct gccccacaca ctgtcattgc 120

gagcccgacg gcaggatgtt gctcagggtg gactgctccg acctggggct ctcggagctg 180

ccttccaacc tcagcgtctt cacctcctac ctagacctca gtatgaacaa catcagtcag 240

ctgctcccga atcccctgcc cagtctccgc ttcctggagg agttacgtct tgcgggaaac 300

gctctgacat acattcccaa gggagcattc actggccttt acagtcttaa agttcttatg 360

ctgcagaata atcagctaag acacgtaccc acagaagctc tgcagaattt gcgaagcctt 420

caatccctgc gtctggatgc taaccacatc agctatgtgc ccccaagctg tttcagtggc 480

ctgcattccc tgaggcacct gtggctggat gacaatgcgt taacagaaat ccccgtccag 540

gcttttagaa gtttatcggc attgcaagcc atgaccttgg ccctgaacaa aatacaccac 600

ataccagact atgcctttgg aaacctctcc agcttggtag ttctacatct ccataacaat 660

agaatccact ccctgggaaa gaaatgcttt gatgggctcc acagcctaga gactttagat 720

ttaaattaca ataaccttga tgaattcccc actgcaatta ggacactctc caaccttaaa 780

gaactaggat ttcatagcaa caatatcagg tcgatacctg agaaagcatt tgtaggcaac 840

ccttctctta ttacaataca tttctatgac aatcccatcc agtttgttgg gagatctgct 900

tttcaacatt tacctgaact aagaacactg actctgaatg gtgcctcaca aataactgaa 960

tttcctgatt taactggaac tgcaaacctg gagagtctga ctttaactgg agcacagatc 1020

tcatctcttc ctcaaaccgt ctgcaatcag ttacctaatc tccaagtgct agatctgtct 1080

tacaacctat tagaagattt acccagtttt tcagtctgcc aaaagcttca gaaaattgac 1140

ctaagacata atgaaatcta cgaaattaaa gttgacactt tccagcagtt gcttagcctc 1200

cgatcgctga atttggcttg gaacaaaatt gctattattc accccaatgc attttccact 1260

ttgccatccc taataaagct ggacctatcg tccaacctcc tgtcgtcttt tcctataact 1320

gggttacatg gtttaactca cttaaaatta acaggaaatc atgccttaca gagcttgata 1380

tcatctgaaa actttccaga actcaaggtt atagaaatgc cttatgctta ccagtgctgt 1440

gcatttggag tgtgtgagaa tgcctataag atttctaatc aatggaataa aggtgacaac 1500

agcagtatgg acgaccttca taagaaagat gctggaatgt ttcaggctca agatgaacgt 1560

gaccttgaag atttcctgct tgactttgag gaagacctga aagcccttca ttcagtgcag 1620

tgttcacctt ccccaggccc cttcaaaccc tgtgaacacc tgcttgatgg ctggctgatc 1680

agaattggag tgtggaccat agcagttctg gcacttactt gtaatgcttt ggtgacttca 1740

acagttttca gatcccctct gtacatttcc cccattaaac tgttaattgg ggtcatcgca 1800

gcagtgaaca tgctcacggg agtctccagt gccgtgctgg ctggtgtgga tgcgttcact 1860

tttggcagct ttgcacgaca tggtgcctgg tgggagaatg gggttggttg ccatgtcatt 1920

ggttttttgt ccatttttgc ttcagaatca tctgttttcc tgcttactct ggcagccctg 1980

gagcgtgggt tctctgtgaa atattctgca aaatttgaaa cgaaagctcc attttctagc 2040

ctgaaagtaa tcattttgct ctgtgccctg ctggccttga ccatggccgc agttcccctg 2100

ctgggtggca gcaagtatgg cgcctcccct ctctgcctgc ctttgccttt tggggagccc 2160

agcaccatgg gctacatggt cgctctcatc ttgctcaatt ccctttgctt cctcatgatg 2220

accattgcct acaccaagct ctactgcaat ttggacaagg gagacctgga gaatatttgg 2280

gactgctcta tggtaaaaca cattgccctg ttgctcttca ccaactgcat cctaaactgc 2340

cctgtggctt tcttgtcctt ctcctcttta ataaacctta catttatcag tcctgaagta 2400

attaagttta tccttctggt ggtagtccca cttcctgcat gtctcaatcc ccttctctac 2460

atcttgttca atcctcactt taaggaggat ctggtgagcc tgagaaagca aacctacgtc 2520

tggacaagat caaaacaccc aagcttgatg tcaattaact ctgatgatgt cgaaaaacag 2580

tcctgtgact caactcaagc cttggtaacc tttaccagct ccagcatcac ttatgacctg 2640

cctcccagtt ccgtgccatc accagcttat ccagtgactg agagctgcca tctttcctct 2700

gtggcatttg tcccatgtct ctaa 2724

<210> 15
<211> 2997
<212> DNA

<213>  Homo sapiens

<220>
<221>  EPHB3
<222>  (1)..(2997)

<400>  15
atggccagag cccgcccgcc gccgccgccg tcgccgccgc cggggcttct gccgctgctc      60

cctccgctgc tgctgctgcc gctgctgctg ctgcccgccg gctgccgggc gctggaagag     120

accctcatgg acacaaaatg ggtaacatct gagttggcgt ggacatctca tccagaaagt     180

gggtgggaag aggtgagtgg ctacgatgag gccatgaatc ccatccgcac ataccaggtg     240

tgtaatgtgc gcgagtcaag ccagaacaac tggcttcgca cggggttcat ctggcggcgg     300

gatgtgcagc gggtctacgt ggagctcaag ttcactgtgc gtgactgcaa cagcatcccc     360

aacatccccg gctcctgcaa ggagaccttc aacctcttct actacgaggc tgacagcgat     420

gtggcctcag cctcctcccc cttctggatg gagaacccct acgtgaaagt ggacaccatt     480

gcacccgatg agagcttctc gcggctggat gccggccgtg tcaacaccaa ggtgcgcagc     540

tttgggccac tttccaaggc tggcttctac ctggccttcc aggaccaggg cgcctgcatg     600

tcgctcatct ccgtgcgcgc cttctacaag aagtgtgcat ccaccaccgc aggcttcgca     660

ctcttccccg agaccctcac tggggcggag cccacctcgc tggtcattgc tcctggcacc     720

tgcatcccta acgccgtgga ggtgtcggtg ccactcaagc tctactgcaa cggcgatggg     780

gagtggatgg tgcctgtggg tgcctgcacc tgtgccaccg gccatgagcc agctgccaag     840

gagtcccagt gccgcccctg tccccctggg agctacaagg cgaagcaggg agaggggccc     900

tgcctcccat gtccccccaa cagccgtacc acctccccag ccgccagcat ctgcacctgc     960

cacaataact tctaccgtgc agactcggac tctgcggaca gtgcctgtac caccgtgcca    1020

tctccacccc gaggtgtgat ctccaatgtg aatgaaacct cactgatcct cgagtggagt    1080

gagccccggg acctgggtgg ccgggatgac ctcctgtaca atgtcatctg caagaagtgc    1140

catggggctg gaggggcctc agcctgctca cgctgtgatg acaacgtgga gtttgtgcct    1200

cggcagctgg gcctgacgga gcgccgggtc cacatcagcc atctgctggc ccacacgcgc    1260

tacacctttg aggtgcaggc ggtcaacggt gtctcgggca agagccctct gccgcctcgt    1320

tatgcggccg tgaatatcac cacaaaccag gctgccccgt ctgaagtgcc cacactacgc    1380

ctgcacagca gctcaggcag cagcctcacc ctatcctggg cacccccaga gcggcccaac    1440

ggagtcatcc tggactacga gatgaagtac tttgagaaga gcgagggcat cgcctccaca    1500

gtgaccagcc agatgaactc cgtgcagctg gacgggcttc ggcctgacgc ccgctatgtg    1560

gtccaggtcc gtgcccgcac agtagctggc tatgggcagt acagccgccc tgccgagttt    1620

gagaccacaa gtgagagagg ctctggggcc cagcagctcc aggagcagct tcccctcatc    1680

gtgggctccg ctacagctgg gcttgtcttc gtggtggctg tcgtggtcat cgctatcgtc    1740

tgcctcagga agcagcgaca cggctctgat tcggagtaca cggagaagct gcagcagtac 1800

attgctcctg gaatgaaggt ttatattgac ccttttacct acgaggaccc taatgaggct 1860

gttcgggagt ttgccaagga gatcgacgtg tcctgcgtca agatcgagga ggtgatcgga 1920

gctggggaat ttggggaagt gtgccgtggt cgactgaaac agcctggccg ccgagaggtg 1980

tttgtggcca tcaagacgct gaaggtgggc tacaccgaga ggcagcggcg ggacttccta 2040

agcgaggcct ccatcatggg tcagtttgat caccccaata taatccggct cgagggcgtg 2100

gtcaccaaaa gtcggccagt tatgatcctc actgagttca tggaaaactg cgccctggac 2160

tccttcctcc ggctcaacga tgggcagttc acggtcatcc agctggtggg catgttgcgg 2220

ggcattgctg ccggcatgaa gtacctgtcc gagatgaact atgtgcaccg cgacctggct 2280

gctcgcaaca tccttgtcaa cagcaacctg gtctgcaaag tctcagactt tggcctctcc 2340

cgcttcctgg aggatgaccc ctccgatcct acctacacca gttccctggg cgggaagatc 2400

cccatccgct ggactgcccc agaggccata gcctatcgga agttcacttc tgctagtgat 2460

gtctggagct acggaattgt catgtgggag gtcatgagct atggagagcg accctactgg 2520

gacatgagca accaggatgt catcaatgcc gtggagcagg attaccggct gccaccaccc 2580

atggactgtc ccacagcact gcaccagctc atgctggact gctgggtgcg ggaccggaac 2640

ctcaggccca aattctccca gattgtcaat accctggaca agctcatccg caatgctgcc 2700

agcctcaagg tcattgccag cgctcagtct ggcatgtcac agcccctcct ggaccgcacg 2760

gtcccagatt acacaacctt cacgacagtt ggtgattggc tggatgccat caagatgggg 2820

cggtacaagg agagcttcgt cagtgcgggg tttgcatctt ttgacctggt ggcccagatg 2880

acggcagaag acctgctccg tattggggtc accctggccg gccaccagaa gaagatcctg 2940

agcagtatcc aggacatgcg gctgcagatg aaccagacgc tgcctgtgca ggtctga 2997

<210> 16
<211> 852
<212> DNA
<213> Homo sapiens

<220>
<221> KLF6
<222> (1)..(852)

<400> 16
atggacgtgc tccccatgtg cagcatcttc caggagctcc agatcgtgca cgagaccggc 60

tacttctcgg cgctgccgtc tctggaggag tactggcaac agacctgcct agagctggaa 120

cgttacctcc agagcgagcc ctgctatgtt tcagcctcag aaatcaaatt tgacagccag 180

gaagatctgt ggaccaaaat cattctggct cgggagaaaa aggaggaatc cgaactgaag 240

atatcttcca gtcctccaga ggacactctc atcagcccga gcttttgtta caacttagag 300

accaacagcc tgaactcaga tgtcagcagc gaatcctctg acagctccga ggaactttct 360

cccacggcca agtttacctc cgaccccatt ggcgaagttt tggtcagctc gggaaaattg 420

agctcctctg tcacctccac gcctccatct tctccggaac tgagcaggga accttctcaa 480

ctgtggggtt gcgtgcccgg ggagctgccc tcgccaggga aggtgcgcag cgggacttcg 540

gggaagccag gtgacaaggg aaatggcgat gcctcccccg acggcaggag gagggtgcac 600

cggtgccact ttaacggctg caggaaagtt tacaccaaaa gctcccactt gaaagcacac 660

cagcggacgc acacaggaga aaagccttac agatgctcat gggaagggtg tgagtggcgt 720

tttgcaagaa gtgatgagtt aaccaggcac ttccgaaagc acaccggggc caagcctttt 780

aaatgctccc actgtgacag gtgtttttcc aggtctgacc acctggccct gcacatgaag 840

aggcacctct ga 852

<210> 17
<211> 888
<212> DNA
<213> Homo sapiens

<220>
<221> CCND1
<222> (1)..(888)

<400> 17
atggaacacc agctcctgtg ctgcgaagtg gaaaccatcc gccgcgcgta ccccgatgcc 60

aacctcctca acgaccgggt gctgcgggcc atgctgaagg cggaggagac ctgcgcgccc 120

tcggtgtcct acttcaaatg tgtgcagaag gaggtcctgc cgtccatgcg gaagatcgtc 180

gccacctgga tgctggaggt ctgcgaggaa cagaagtgcg aggaggaggt cttcccgctg 240

gccatgaact acctggaccg cttcctgtcg ctggagcccg tgaaaaagag ccgcctgcag 300

ctgctggggg ccacttgcat gttcgtggcc tctaagatga aggagaccat ccccctgacg 360

gccgagaagc tgtgcatcta caccgacaac tccatccggc ccgaggagct gctgcaaatg 420

gagctgctcc tggtgaacaa gctcaagtgg aacctggccg caatgacccc gcacgatttc 480

attgaacact tcctctccaa aatgccagag gcggaggaga acaaacagat catccgcaaa 540

cacgcgcaga ccttcgttgc cctctgtgcc acagatgtga agttcatttc caatccgccc 600

tccatggtgg cagcggggag cgtggtggcc gcagtgcaag gcctgaacct gaggagcccc 660

aacaacttcc tgtcctacta ccgcctcaca cgcttcctct ccagagtgat caagtgtgac 720

ccggactgcc tccgggcctg ccaggagcag atcgaagccc tgctggagtc aagcctgcgc 780

caggcccagc agaacatgga ccccaaggcc gccgaggagg aggaagagga ggaggaggag 840

gtggacctgg cttgcacacc caccgacgtg cgggacgtgg acatctga 888

<210> 18
<211> 303

<212>  DNA
<213>  Homo sapiens


<220>
<221>  DEFA6
<222>  (1)..(303)

<400>  18
atgagaaccc tcaccatcct cactgctgtt ctcctcgtgg ccctccaggc caaggctgag      60

ccactccaag ctgaggatga tccactgcag gcaaaagctt atgaggctga tgcccaggag     120

cagcgtgggg caaatgacca ggactttgcc gtctcctttg cagaggatgc aagctcaagt     180

cttagagctt tgggctcaac aagggctttc acttgccatt gcagaaggtc ctgttattca     240

acagaatatt cctatgggac ctgcactgtc atgggtatta accacagatt ctgctgcctc     300

tga                                                                   303


<210>  19
<211>  1725
<212>  DNA
<213>  Homo sapiens


<220>
<221>  FZD7
<222>  (1)..(1725)

<400>  19
atgcgggacc ccggcgcggc cgctccgctt tcgtccctgg gcctctgtgc cctggtgctg      60

gcgctgctgg gcgcactgtc cgcgggcgcc ggggcgcagc cgtaccacgg agagaagggc     120

atctccgtgc cggaccacgg cttctgccag cccatctcca tcccgctgtg cacggacatc     180

gcctacaacc agaccatcct gcccaacctg ctgggccaca cgaaccaaga ggacgcgggc     240

ctcgaggtgc accagttcta cccgctggtg aaggtgcagt gttctcccga actccgcttt     300

ttcttatgct ccatgtatgc gcccgtgtgc accgtgctcg atcaggccat cccgccgtgt     360

cgttctctgt gcgagcgcgc ccgccagggc tgcgaggcgc tcatgaacaa gttcggcttc     420

cagtggcccg agcggctgcg ctgcgagaac ttcccggtgc acggtgcggg cgagatctgc     480

gtgggccaga acacgtcgga cggctccggg ggcccaggcg gcggccccac tgcctaccct     540

accgcgccct acctgccgga cctgcccttc accgcgctgc ccccgggggc ctcagatggc     600

agggggcgtc ccgccttccc cttctcatgc ccccgtcagc tcaaggtgcc cccgtacctg     660

ggctaccgct tcctgggtga gcgcgattgt ggcgccccgt gcgaaccggg ccgtgccaac     720

ggcctgatgt actttaagga ggaggagagg cgcttcgccc gcctctgggt gggcgtgtgg     780

tccgtgctgt gctgcgcctc gacgctcttt accgttctca cctacctggt ggacatgcgg     840

cgcttcagct acccagagcg gcccatcatc ttcctgtcgg gctgctactt catggtggcc     900

gtggcgcacg tggccggctt ccttctagag gaccgcgccg tgtgcgtgga gcgcttctcg     960

gacgatggct accgcacggt ggcgcagggc accaagaagg agggctgcac catcctcttc 1020

atggtgctct acttcttcgg catggccagc tccatctggt gggtcattct gtctctcact 1080

tggttcctgg cggccggcat gaagtggggc cacgaggcca tcgaggccaa ctcgcagtac 1140

ttccacctgg ccgcgtgggc cgtgcccgcc gtcaagacca tcactatcct ggccatgggc 1200

caggtagacg gggacctgct gagcggggtg tgctacgttg gcctctccag tgtggacgcg 1260

ctgcggggct tcgtgctggc gcctctgttc gtctacctct tcataggcac gtccttcttg 1320

ctggccggct tcgtgtccct cttccgtatc cgcaccatca tgaaacacga cggcaccaag 1380

accgagaagc tggagaagct catggtgcgc atcggcgtct tcagcgtgct ctacacagtg 1440

cccgccacca tcgtcctggc ctgctacttc tacgagcagg ccttccgcga gcactgggag 1500

cgcacctggc tcctgcagac gtgcaagagc tatgccgtgc cctgcccgcc cggccacttc 1560

ccgcccatga gccccgactt caccgtcttc atgatcaagt acctgatgac catgatcgtc 1620

ggcatcacca ctggcttctg gatctggtcg ggcaagaccc tgcagtcgtg gcgccgcttc 1680

taccacagac ttagccacag cagcaagggg gagactgcgg tatga 1725

<210> 20
<211> 1413
<212> DNA
<213> Homo sapiens

<220>
<221> NKD1
<222> (1)..(1413)

<400> 20
atggggaaac ttcactccaa gccggccgcc gtgtgcaagc gcagggagag cccggaaggt 60

gacagcttcg ccgtgagcgc tgcctgggct cggaagggca tcgaggagtg gatcgggaga 120

cagcgctgcc cgggcggtgt ctcgggaccc cgacagctgc ggttggcggg caccataggc 180

cgaagcaccc gggagctcgt gggcgacgtg ttgagagaca cgctcagcga ggaagaggag 240

gacgactttc ggctggaagt ggccctgcct cctgagaaga ctgacgggct gggcagcgga 300

gatgagaaga agatggagag agtgagcgaa ccctgcccag gctccaagaa gcagctgaag 360

tttgaagagc tccagtgcga cgtgtccatg gaggaggaca gccggcagga gtggaccttc 420

accctgtatg actttgacaa caacggcaag gtcacccgag aggacatcac cagcttgctg 480

cacaccatct atgaggtggt ggactcctct gtcaaccact ccccaacatc cagcaagatg 540

ctgcgggtaa agctcaccgt ggcccccgat ggcagccaga gcaagaggag cgtccttgtc 600

aatcaggctg acctgcagag cgcaaggccc cgagcagaga ccaagcccac tgaggacctg 660

cggagctggg agaagaagca gcgagccccg ctcaggttcc agggtgacag ccgcctggag 720

cagtctggct gctaccacca ttgcgtagat gagaacatcg agaggagaaa ccactactta 780

gatctcgccg ggatagaaaa ctacacgtcc caatttgggc ctggctcccc ttccgtggcc 840

cagaagtcag aactgccccc ccgcacctcc aatcccactc gatctcgctc ccatgagccg 900

gaagccatcc acatcccaca ccgaaagccc caaggcgtgg acccggcctc cttccacttc 960

cttgacaccc caatcgccaa ggtctcagag ctccagcaac ggctccgggg cacccaggac 1020

gggagcaagc actttgtgag gtcccccaag gcccagggca agagtgtggg tgtgggccac 1080

gtggccagag gggcaagaaa caagccccct ctgggacccg ccatccctgc ggtgtccccc 1140

tccgcccacc tggctgccag cccggccctc ctcccctccc tagcccccct cgggcacaag 1200

aagcacaagc accgagccaa ggagagccag cagggctgcc ggggcctgca ggcaccactg 1260

gcctcaggtg gccctgtcct ggggcgggag cacctgcggg agctgcccgc cttggtggtg 1320

tatgagagcc aggccgggca gccggtccag agacatgagc accaccacca ccatgaacat 1380

caccaccatt accaccactt ctaccagaca tag 1413

<210> 21
<211> 1320
<212> DNA
<213> Homo sapiens

<220>
<221> OAT
<222> (1)..(1320)

<400> 21
atgttttcca aactagcaca tttgcagagg tttgctgtac ttagtcgcgg agttcattct 60

tcagtggctt ctgctacatc tgttgcaact aaaaaaacag tccaaggccc tccaacctct 120

gatgacattt ttgaaaggga atataagtat ggtgcacaca actaccatcc tttacctgta 180

gccctggaga gaggaaaagg tatttactta tgggatgtag aaggcagaaa atattttgac 240

ttcctgagtt cttacagtgc tgtcaaccaa gggcattgtc accccaagat tgtgaatgct 300

ctgaagagtc aagtggacaa attgacctta acatctagag ctttctataa taacgtactt 360

ggtgaatatg aggagtatat tactaaactt ttcaactacc acaaagttct tcctatgaat 420

acaggagtgg aggctggaga gactgcctgt aaactagctc gtaagtgggg ctataccgtg 480

aagggcattc agaaatacaa agcaaagatt gtttttgcag ctgggaactt ctggggtagg 540

acgttgtctg ctatctccag ttccacagac ccaaccagtt acgatggttt tggaccattt 600

atgccgggat tcgacatcat tccctataat gatctgcccg cactggagcg tgctcttcag 660

gatccaaatg tggctgcgtt catggtagaa ccaattcagg gtgaagcagg cgttgttgtt 720

ccggatccag gttacctaat gggagtgcga gagctctgca ccaggcacca ggttctcttt 780

attgctgatg aaatacagac aggattggcc agaactggta gatggctggc tgttgattat 840

gaaaatgtca gacctgatat agtcctcctt ggaaaggccc tttctggggg cttataccct 900

gtgtctgcag tgctgtgtga tgatgacatc atgctgacca ttaagccagg ggagcatggg 960

tccacatacg gtggcaatcc actaggctgc cgagtggcca tcgcagccct tgaggtttta 1020

gaagaagaaa accttgctga aaatgcagac aaattgggca ttatcttgag aaatgaactc 1080

atgaagctac cttctgatgt tgtaactgcc gtaagaggaa aaggattatt aaacgctatt 1140

gtcattaaag aaaccaaaga ttgggatgct tggaaggtgt gtctacgact tcgagataat 1200

ggacttctgg ccaagccaac ccatggcgac attatcaggt ttgcgcctcc gctggtgatc 1260

aaggaggatg agcttcgaga gtccattgaa attattaaca agaccatctt gtctttctga 1320

<210> 22
<211> 13767
<212> DNA
<213> Homo sapiens

<220>
<221> FAT1
<222> (1)..(13767)

<400> 22
   atggggagac atttggcttt gctcctgctt ctgctccttc tcttccaaca ttttggagac 60
   agtgatggca gccaacgact tgaacagact cctctgcagt ttacacacct cgagtacaac 120
   gtcaccgtgc aggagaactc tgcagctaag acttatgtgg ggcatcctgt caagatgggt 180
   gtttacatta cacatccagc gtgggaagta aggtacaaaa ttgtttccgg agacagtgaa 240
   aacctgttca aagctgaaga gtacattctc ggagactttt gctttctaag aataaggacc 300
   aaaggaggaa atacagctat tcttaataga gaagtgaagg atcactacac attgatagtg 360
   aaagcacttg aaaaaaatac taatgtggag gcgcgaacaa aggtcagggt gcaggtgctg 420
   gatacaaatg acttgagacc gttattctca cccacctcat acagcgtttc tttacctgaa 480
   aacacagcta taaggaccag tatcgcaaga gtcagcgcca cggatgcaga cataggaacc 540
   aacggggaat tttactacag ttttaaagat cgaacagata tgtttgctat tcacccaacc 600
   agtggtgtga tagtgttaac tggtagactt gattacctag agaccaagct ctatgagatg 660
   gaaatcctcg ctgcggaccg tggcatgaag ttgtatggga gcagtggcat cagcagcatg 720
   gccaagctaa cggtgcacat cgaacaggcc aatgaatgtg ctccggtgat aacagcagtg 780
   acattgtcac catcagaact ggacagggac ccagcatatg caattgtgac agtggatgac 840
   tgcgatcagg gtgccaatgg tgacatagca tctttaagca tcgtggcagg tgaccttctc 900
   cagcagttta gaacagtgag gtcctttcca gggagtaagg agtataaagt caaagccatc 960
   ggtggcattg attgggacag tcatcctttc ggctacaatc tcacactaca ggctaaagat 1020
   aaaggaactc cgccccagtt ctcttctgtt aaagtcattc acgtgacttc tccacagttc 1080
   aaagccgggc cagtcaagtt tgaaaaggat gtttacagag cagaaataag tgaatttgct 1140
   cctcccaaca cacctgtggt catggtaaag gccattcctg cttattccca tttgaggtat 1200
   gtttttaaaa gtacacctgg aaaagctaaa ttcagtttaa attacaacac tggtctcatt 1260
   tctattttag aaccagttaa aagacagcag gcagcccatt ttgaacttga agtaacaaca 1320
   agtgacagaa aagcgtccac caaggtcttg gtgaaagtct taggtgcaaa tagcaatccc 1380
   cctgaattta cccagacagc gtacaaagct gcttttgatg agaacgtgcc cattggtact 1440
   actgtcatga gcctgagtgc cgtagaccct gatgagggtg agaacgggta cgtgacatac 1500
   agtatcgcaa atttaaatca tgtgccgttt gcgattgacc atttcactgg tgccgtgagt 1560
   acgtcagaaa acctggacta cgaactgatg cctcgggttt atactctgag gattcgtgca 1620
   tcagactggg gcttgccgta ccgccgggaa gtcgaagtcc ttgctacaat tactctcaat 1680
   aacttgaatg acaacacacc tttgtttgag aaaataaatt gtgaagggac aattcccaga 1740
   gatctaggcg tgggagagca aataaccact gtttctgcta ttgatgcaga tgaacttcag 1800
   ttggtacagt atcagattga agctggaaat gaactggatt tctttagttt aaaccccaac 1860
   tcgggggtat tgtcattaaa gcgatcgcta atggatggct taggtgcaaa ggtgtctttc 1920
   cacagtctga gaatcacagc tacagatgga gaaaattttg ccacaccatt atatatcaac 1980
   ataacagtgg ctgccagtca caagctggta aacttgcagt gtgaagagac tggtgttgcc 2040
   aaaatgctgg cagagaagct cctgcaggca aataaattac acaaccaggg agaggtggag 2100
   gatattttct tcgattctca ctctgtcaat gctcacatac cgcagtttag aagcactctt 2160
   ccgactggta ttcaggtaaa ggaaaaccag cctgtgggtt ccagtgtaat tttcatgaac 2220
   tccactgacc ttgacactgg cttcaatgga aaactggtct atgctgtttc tggaggaaat 2280
   gaggatagtt gcttcatgat tgatatggaa acaggaatgc tgaaaatttt atctcctctt 2340
   gaccgtgaaa caacagacaa atacaccctg aatattaccg tctatgacct tgggataccc 2400
   cagaaggctg cgtggcgtct tctacatgtc gtggttgtcg atgccaatga taatccaccc 2460
   gagtttttac aggagagcta ttttgtggaa gtgagtgaag acaaggaggt acatagtgaa 2520
   atcatccagg ttgaagccac agataaagac ctggggccca acggacacgt gacgtactca 2580
   attgttacag acacagacac attttcaatt gacagcgtga cgggtgttgt taacatcgca 2640
   cgccctctgg atcgagagct gcagcatgag cactccttaa agattgaggc cagggaccaa 2700
   gccagagaag agcctcagct gttctccact gtcgttgtga aagtatcact agaagatgtt 2760
   aatgacaacc cacctacatt tattccacct aattatcgtg tgaaagtccg agaggatctt 2820
   ccagaaggaa ccgtcatcat gtggttagaa gcccacgatc ctgatttagg tcagtctggt 2880
   caggtgagat acagccttct ggaccacgga gaaggaaact tcgatgtgga taaactcagt 2940
   ggagcagtta ggatcgtcca gcagttggac tttgagaaga agcaagtgta taatctcact 3000
   gtgagggcca aagacaaggg aaagccagtt tctctgtctt ctacttgcta tgttgaagtt 3060
   gaggtggttg atgtgaatga gaacctgcac ccacccgtgt tttccagctt tgtggaaaag 3120
   gggacagtga aagaagatgc acctgttggt tcattggtaa tgacggtgtc ggctcatgat 3180
   gaggacgcca gaagagatgg ggagatccga tactccatta gagatggctc tggcgttggt 3240
   gttttcaaaa taggtgaaga gacaggtgtc atagagacgt cagatcgact ggaccgtgaa 3300
   tcgacctccc attattggct aacagtcttt gcaaccgatc agggtgtcgt gcctctttca 3360
   tcgttcatag agatctacat agaggttgag gatgtcaatg acaatgcacc acagacatca 3420
   gagcctgttt attacccaga aatcatggaa aattctccta aagatgtatc tgtggtccag 3480
   atcgaggcat ttgatccaga ttcgagctct aatgacaagc tcatgtacaa aattacaagt 3540
   ggaaatccac aaggattctt ttcaatacat cctaaaacag gtctcatcac aactacgtca 3600
   aggaagctag accgagaaca gcaagatgaa cacatattag aggttactgt gacagacaat 3660
   ggtagtcccc ccaaatcaac cattgcaaga gtcattgtga aaatccttga tgaaaatgac 3720
   aacaaacctc agtttctgca aaagttctac aaaatcagac tccctgagcg ggaaaagcca 3780
   gaccgagaaa gaaatgccag acgggagccg ctctatcacg tcatagccac cgacaaggat 3840
   gagggcccca atgcagaaat ctcctacagc atcgaagacg ggaatgagca tggcaaattt 3900
   ttcatcgaac cgaaaactgg agtggtttcg tccaagaggt tttcagcagc tggagaatat 3960
   gatattcttt caattaaggc agttgacaat ggtcgccctc aaaagtcatc aaccaccaga 4020
   ctccatattg aatggatctc caagcccaaa ccgtccctgg agcccatttc atttgaagaa 4080
   tcatttttta cctttactgt gatggaaagt gaccccgttg ctcacatgat tggagtaata 4140
   tctgtggagc ctcctggcat acccctttgg tttgacatca ctggtggcaa ctacgacagt 4200
   cacttcgatg tggacaaggg aactggaacc atcattgttg ccaaacctct tgatgcagaa 4260
   cagaagtcaa actacaacct cacagtcgag gctacagatg gaaccaccac tatcctcact 4320
   caggtattca tcaaagtaat agacacaaat gaccatcgtc ctcagttttc tacatcaaag 4380
   tatgaagttg ttattcctga agatacagcg ccagaaacag aaattttgca aatcagtgct 4440
   gtggatcagg atgagaaaaa caaactaatc tacactctgc agagcagtag agatccactg 4500
   agtctcaaga aatttcgtct tgatcctgca accggctctc tctatacttc tgagaaactg 4560
   gatcatgaag ctgttcacca gcacaccctc acggtcatgg tacgagatca agatgtgcct 4620
   gtaaaacgca actttgcaag gattgtggtc aatgtcagcg acacgaatga ccacgccccg 4680
   tggttcaccg cttcctccta caaagggcgg gtttatgaat cggcagccgt tggctcagtt 4740
   gtgttgcagg tgacggctct ggacaaggac aaagggaaaa atgctgaagt gctgtactcg 4800
   atcgagtcag gaaatattgg aaattctttt atgattgatc ctgtcttggg ctctattaaa 4860
   actgccaaag aattagatcg aagtaaccaa gcggagtatg atttaatggt aaaagctaca 4920
   gataagggca gtccaccaat gagtgaaata acttctgtgc gtatctttgt cacaattgct 4980
   gacaacgcct ctccgaagtt tacatcaaaa gaatattctg ttgaacttag tgaaactgtc 5040
   agcattggga gtttcgttgg gatggttaca gcccatagtc aatcatcagt ggtgtatgaa 5100
   ataaaagatg gaaatacagg tgatgctttt gatattaatc cacattctgg aactatcatc 5160
   actcagaaag ccctggactt tgaaactttg cccatttaca cattgataat acaaggaact 5220
   aacatggctg gtttgtccac taatacaacg gttctagttc acttgcagga tgagaatgac 5280
   aacgcgccag tttttatgca ggcagaatat acaggactca ttagtgaatc agcctcaatt 5340
   aacagcgtgg tcctaacaga caggaatgtc ccactggtga ttcgagcagc tgatgctgat 5400
   aaagactcaa atgctttgct tgtatatcac attgttgaac catctgtaca cacatatttt 5460
   gctattgatt ctagcactgg tgctattcat acagtactaa gtctggacta tgaagaaaca 5520
   agtatttttc actttaccgt ccaagtgcat gacatgggaa ccccacgttt atttgctgag 5580
   tatgcagcga atgtaacagt acatgtaatt gacattaatg actgcccccc tgtgtttgcc 5640
   aagccattat atgaagcatc tcttttgtta ccaacataca aaggagtaaa agtcatcaca 5700
   gtaaatgcta cagatgctga ttcaagtgca ttctcacagt tgatttactc catcaccgaa 5760
   ggcaacatcg gggagaagtt ttctatggac tacaagactg gtgctctcac tgtccaaaac 5820
   acaactcagt taagaagccg ctacgagcta accgttagag cttccgatgg cagatttgcc 5880
   ggccttacct ctgtcaaaat taatgtgaaa gaaagcaaag aaagtcacct aaagtttacc 5940
   caggatgtct actctgcggt agtgaaagag aattccaccg aggccgaaac attagctgtc 6000
   attactgcta ttgggaatcc aatcaatgag cctttgtttt atcacatcct caacccagat 6060
   cgcagattta aaataagccg cacttcagga gttctgtcaa ccactggcac gcccttcgat 6120
   cgtgagcagc aggaggcgtt tgatgtggtt gtagaagtga cagaggaaca taagccttct 6180
   gcagtggccc acgttgtcgt gaaggtcatt gtagaagacc aaaatgataa tgcgccggtg 6240
   tttgtcaacc ttccctacta cgccgttgtt aaagtggaca ctgaggtggg ccatgtcatt 6300
   cgctatgtca ctgctgtaga cagagacagt ggcagaaacg gggaagtgca ttactacctc 6360
   aaggaacatc atgaacactt tcaaattgga cccttgggtg aaatttcact gaaaaagcaa 6420
   tttgagcttg acaccttaaa taaagaatat cttgttacag tggttgcaaa agatggaggg 6480
   aacccggcct tttcagcgga agttatcgtt ccgatcactg tcatgaataa agccatgcct 6540
   gtgtttgaaa aacctttcta cagtgcagag attgcagaga gcatccaggt gcacagccct 6600
   gtggtccacg tgcaggctaa cagcccggaa ggcctgaaag tgttctacag catcacagac 6660
   ggagaccctt tcagccagtt cactattaac ttcaatactg gagttatcaa tgtcatagct 6720
   cctctggact ttgaggccca cccggcatat aagctgagca tacgcgcaac tgactccttg 6780
   acgggcgctc atgctgaagt atttgtggac atcatagtag acgacatcaa tgataaccct 6840
   cctgtgtttg ctcagcagtc ttatgcggtg accctgtctg aggcatctgt aattggaacg 6900
   tctgttgttc aagttagagc caccgattct gattcagaac caaatagagg aatctcatac 6960
   cagatgtttg ggaatcacag caagagtcat gatcattttc atgtagacag cagcactggc 7020
   ctcatctcac tactcagaac cctggattac gagcagtccc ggcagcacac gatttttgtg 7080
   agggcagttg atggtggtat gcccacgctg agcagtgatg tgattgtcac ggtggacgtt 7140
   accgacctca atgataatcc accactcttt gaacaacaga tttatgaagc cagaattagc 7200
   gagcacgccc ctcatgggca tttcgtgacc tgtgtaaaag cctatgatgc agacagttca 7260
   gacatagaca agttgcagta ttccattctg tctggcaatg atcataaaca ttttgtcatt 7320
   gacagtgcaa cagggattat caccctctca aacctgcacc ggcacgccct gaagccattt 7380
   tacagtctta acctgtcagt gtctgatgga gtttttagaa gttccaccca ggttcatgta 7440
   actgtaattg gaggcaattt gcacagtcct gctttccttc agaacgaata tgaagtggaa 7500
   ctagctgaaa acgctcccct acataccctg gtgatggagg tgaaaactac ggatggggat 7560
   tctggtattt atggtcacgt tacttaccat attgtaaatg actttgccaa agacagattt 7620
   tacataaatg agagaggaca gatatttact ttggaaaaac ttgatcgaga aaccccggcg 7680
   gagaaagtga tctcagtccg tttaatggct aaggatgctg gaggaaaagt tgctttctgc 7740
   accgtgaatg tcatccttac agatgacaat gacaatgcac cacaatttcg agcaaccaaa 7800
   tacgaagtga atatcgggtc cagtgctgct aaaggacttc cagtcgttaa agttcttgca 7860
   agtgatgccg atgagggctc caatgccgac atcacctatg ccattgaagc agactctgaa 7920
   agtgtaaaag agaatttgga aattaacaaa ctgtccggcg taatcactac aaaggagagc 7980
   ctcattggct tggaaaatga attcttcact ttctttgtta gagctgtgga taatgggtct 8040
   ccatcaaaag aatctgttgt tcttgtctat gttaaaatcc ttccaccgga aatgcagctt 8100
   ccaaaatttt cagaaccttt ctataccttt acagtgtcag aggacgtgcc tattggaaca 8160
   gagatagatc tcatccgagc agaacatagt gggactgttc tttacagcct ggtcaaaggg 8220
   aatactccag aaagcaatag ggatgagtcc tttgtgattg acagacagag cgggagactg 8280
   aagttggaga agagtcttga tcatgagaca actaagtggt atcagttttc catactggcc 8340
   aggtgcactc aagatgacca tgagatggtg gcttctgtag atgttagtat ccaagtgaaa 8400
   gatgcaaatg acaacagccc ggtctttgaa tctagtccat atgaggcatt cattgttgaa 8460
   aacctgccag ggggaagtag agtaattcag atcagggcat ctgatgctga ctcaggaacc 8520
   aacggccaag ttatgtatag cctggatcag tcacaaagtg tggaagtcat tgaatccttt 8580
   gccattaaca tggaaacagg ctggattaca actttaaagg aacttgacca tgaaaagaga 8640
   gacaattacc agattaaagt ggttgcatca gatcatggtg aaaagatcca gctatcctcc 8700
   acagccattg tggatgttac cgtcaccgat gtcaacgata gtccaccacg attcacggcc 8760
   gagatctata aagggactgt gagtgaggat gacccccaag gtggggtgat tgccatctta 8820
   agtaccacgg atgctgattc tgaagagatc aacagacaag ttacatattt cataacagga 8880
   ggggatcctt taggacagtt tgccgttgaa actatacaga atgaatggaa ggtatatgtg 8940
   aagaaacctc tagacaggga aaaaagggac aattaccttc ttactatcac ggcaactgat 9000
   ggcaccttct catcaaaagc gatagttgaa gtgaaagttc tggatgcaaa tgacaacagt 9060
   ccagtttgtg aaaagacttt atattcagac actattcctg aagacgtcct tcctggaaaa 9120
   ttgatcatgc agatctctgc tacagacgca gacatccgct ctaacgctga aattacttac 9180
   acgttattgg gttcaggtgc agaaaaattc aaactaaatc cagacacagg tgaactgaaa 9240
   acgtcaaccc cccttgatcg tgaggagcaa gctgtttatc atcttctcgt cagggccaca 9300
   gatggaggag gaagattctg ccaagccagt attgtgctca cgctagaaga tgtgaacgat 9360
   aacgcccccg aattctctgc cgatccttat gccatcaccg tgtttgaaaa cacagagccg 9420
   ggaacgctgc tgacaagagt gcaggccaca gatgccgacg caggattaaa tcggaagatt 9480
   ttatactcac tgattgactc tgctgatggg cagttctcca ttaacgaatt atctggaatt 9540
   attcagttag aaaaaccttt ggacagagaa ctccaggcag tatacaccct ctctttgaaa 9600
   gctgtggatc aaggcttgcc aaggaggctg actgccactg gcactgtgat tgtatcagtt 9660
   cttgacataa atgacaaccc ccctgtgttt gagtaccgtg aatatggtgc caccgtgtct 9720
   gaggacattc ttgttggaac tgaagttctt caagtgtatg cagcaagtcg ggatattgaa 9780
   gcaaatgcag aaatcaccta ctcaataata agtggaaatg aacatgggaa attcagcata 9840
   gattctaaaa caggggccgt atttatcatt gagaatctgg attatgagag ctctcatgag 9900
   tattacctaa cagtagaggc cactgatgga ggcacgcctt cactgagcga cgttgccact 9960
   gtgaacgtta atgtaacaga tatcaacgat aatacccctg tgttcagcca agacacctac 10020
   acgacagtca tcagtgaaga tgccgttctt gagcagtctg tcatcacggt tatggccgat 10080
   gatgccgatg gaccttccaa cagccacatc cactactcaa ttatagatgg caaccaagga 10140
   agctcgttca caattgaccc cgtcagggga gaagtcaaag tgaccaaact tctcgaccga 10200
   gaaacgattt caggttacac gctcacggtt caagcttctg ataatggcag tccacccaga 10260
   gtcaacacga cgaccgtgaa catcgatgtg tccgatgtca atgacaacgc gcccgtcttc 10320
   tccaggggaa actacagtgt cattatccag gaaaataagc cagtgggctt cagcgtgctg 10380
   cagctggtag taacagatga ggattcttcc cataacggtc cacccttctt ctttactatt 10440
   gtaactggaa atgatgagaa ggcttttgaa gttaacccgc aaggagtcct cctgacatca 10500
   tctgccatca agaggaagga gaaagatcat tacttactgc aggtgaaggt ggcagataat 10560
   ggaaagcctc agttgtcatc tttgacatac attgacatta gggtaattga ggagagcatc 10620
   tatccgcctg cgattttgcc cctggagatt ttcatcacct cttctggaga agaatactca 10680
   ggtggcgtca ttgggaagat ccatgccaca gaccaggacg tgtatgatac tctaacctac 10740
   agtctcgacc ctcagatgga caacctgttc tctgtttcca gcacaggggg caagctgata 10800
   gcacacaaaa agctagacat agggcaatac cttctcaatg tcagcgtaac agatgggaag 10860
   ttcacgacgg tggccgacat cacagtgcat atcagacaag tcacacagga gatgttgaac 10920
   cacaccatcg cgatccgctt tgccaacctc actccggaag aattcgttgg tgactactgg 10980
   cgcaacttcc agcgagcttt acggaacatc ctgggtgtga ggaggaacga catacagatt 11040
   gttagtttgc agtcctctga acctcaccca catctggacg tcttactttt tgtagagaaa 11100
   ccaggtagtg ctcagatctc aacaaaacaa cttctgcaca agattaactc ttccgtgact 11160
   gacattgagg aaatcattgg agttaggata ctgaatgtat tccagaaact ctgcgcggga 11220
   ctggactgcc cctggaagtt ctgcgatgaa aaggtgtctg tggatgaaag tgtgatgtca 11280
   acacacagca cagccagact gagttttgtg actccccgcc accacagggc agcggtgtgt 11340
   ctctgcaaag agggaaggtg cccacctgtc caccatggct gtgaagatga tccgtgccct 11400
   gagggatccg aatgtgtgtc tgatccctgg gaggagaaac acacctgtgt ctgtcccagc 11460
   ggcaggtttg gtcagtgccc agggagttca tctatgacac tgactggaaa cagctacgtg 11520
   aaataccgtc tgacggaaaa tgaaaacaaa ttagagatga aactgaccat gaggctcaga 11580
   acatattcca cgcatgcggt tgtcatgtat gctcgaggaa ctgactatag catcttggag 11640
   attcatcatg gaaggctgca gtacaagttt gactgtggaa gtggccctgg aattgtctct 11700
   gttcagagca ttcaggtcaa tgatgggcag tggcacgcag tggccctgga agtgaatgga 11760
   aactatgctc gcttggttct agaccaagtt catactgcat cgggcacagc cccagggact 11820
   ctgaaaaccc tgaacctgga taactatgtg ttttttggtg gccacatccg tcagcaggga 11880
   acaaggcatg gaagaagtcc tcaagttggt aatggtttca ggggttgtat ggactccatt 11940
   tatttgaatg ggcaggagct ccctttaaac agcaaaccca gaagctatgc acacatcgaa 12000
   gagtcggtgg atgtatctcc aggctgcttc ctgacggcca cggaagactg cgccagcaac 12060
   ccttgccaga atggaggcgt ttgcaatccg tcacctgctg gaggttatta ctgcaaatgc 12120
   agtgccttgt acatagggac ccactgtgag ataagcgtca atccgtgttc ctccaagcca 12180
   tgcctctatg ggggcacgtg tgttgtcgac aacggaggct ttgtttgcca gtgtagagga 12240
   ttatatactg gtcagaggtg tcagcttagt ccatactgca aagatgaacc ctgtaagaat 12300
   ggcggaacat gctttgacag tttggatggc gccgtttgtc agtgtgattc gggttttagg 12360
   ggagaaaggt gtcagagtga tatcgacgag tgctctggaa acccttgcct gcacggggcc 12420
   ctctgtgaga acacgcacgg ctcctatcac tgcaactgca gccacgagta caggggacgt 12480
   cactgcgagg atgctgcgcc caaccagtat gtgtccacgc cgtggaacat tgggttggcg 12540
   gaaggaattg gaatcgttgt gtttgttgca gggatatttt tactggtggt ggtgtttgtt 12600
   ctctgccgta agatgattag tcggaaaaag aagcatcagg ctgaacctaa agacaagcac 12660
   ctgggacccg ctacggcttt cttgcaaaga ccgtattttg attccaagct aaataagaac 12720
   atttactcag acataccacc ccaggtgcct gtccggccta tttcctacac cccgagtatt 12780
   ccaagtgact caagaaacaa tctggaccga aattccttcg aaggatctgc tatcccagag 12840
   catcccgaat tcagcacttt taaccccgag tctgtgcacg ggcaccgaaa agcagtggcg 12900
   gtctgcagcg tggcgccaaa cctgcctccc ccaccccctt caaactcccc ttctgacagc 12960
   gactccatcc agaagcctag ctgggacttt gactatgaca caaaagtggt ggatcttgat 13020
   ccctgtcttt ccaagaagcc tctagaggaa aagccttccc agccatacag tgcccgggaa 13080
   agcctgtctg aagtgcagtc tctgagctcc ttccagtccg aatcgtgcga tgacaatggg 13140
   tatcactggg atacatcaga ttggatgcca agcgttcctc tgccggacat acaagagttc 13200
   cccaactatg aggtgattga tgagcagaca cccctgtact cagcagatcc aaacgccatc 13260
   gatacggact attaccctgg aggctacgac atcgaaagtg attttcctcc acccccagaa 13320
   gacttccccg cagctgatga gctaccaccg ttaccgcccg aattcagcaa tcagtttgaa 13380
   tccatccacc ctcctagaga catgcctgcc gcgggtagct tgggttcttc atcaagaaac 13440
   cggcagaggt tcaacttgaa tcagtatttg cccaattttt atcccctcga tatgtctgaa 13500
   cctcaaacaa aaggcactgg tgagaatagt acttgtagag aaccccatgc cccttacccg 13560
   ccagggtatc aaagacactt cgaggcgccc gctgtcgaga gcatgcccat gtctgtgtac 13620
   gcctccaccg cctcctgctc tgacgtgtca gcctgctgcg aagtggagtc cgaggtcatg 13680
   atgagtgact atgagagcgg ggacgacggc cacttcgaag aggtgacgat cccgcccctg 13740
   gattcccagc agcacacgga agtctga 13767

<210> 23
<211> 1161
<212> DNA
<213> Homo sapiens

<220>
<221> LEF1
<222> (1)..(1161)

<400> 23
atgccccaac tctccggagg aggtggcggc ggcggggggg acccggaact ctgcgccacg 60

gacgagatga tccccttcaa ggacgagggc gatcctcaga aggaaaagat cttcgccgag 120

atcagtcatc ccgaagagga aggcgattta gctgacatca agtcttcctt ggtgaacgag 180

tctgaaatca tcccggccag caacggacac gaggtggcca gacaagcaca aacctctcag 240

gagccctacc acgacaaggc cagagaacac cccgatgacg gaaagcatcc agatggaggc 300

ctctacaaca agggaccctc ctactcgagt tattccgggt acataatgat gccaaatatg 360

aataacgacc catacatgtc aaatggatct ctttctccac ccatcccgag aacatcaaat 420

aaagtgcccg tggtgcagcc atcccatgcg gtccatcctc tcacccccct catcacttac 480

agtgacgagc acttttctcc aggatcacac ccgtcacaca tcccatcaga tgtcaactcc 540

aaacaaggca tgtccagaca tcctccagct cctgatatcc ctacttttta tcccttgtct 600

ccgggtggtg ttggacagat caccccacct cttggctggt tttcccatca tatgattccc 660

ggtcctcctg gtccccacac aactggcatc cctcatccag ctattgtaac acctcaggtc 720

aaacaggaac atccccacac tgacagtgac ctaatgcacg tgaagcctca gcatgaacag 780

agaaaggagc aggagccaaa aagacctcac attaagaagc ctctgaatgc ttttatgtta 840

tacatgaaag aaatgagagc gaatgtcgtt gctgagtgta ctctaaaaga aagtgcagct 900

atcaaccaga ttcttggcag aaggtggcat gccctctccc gtgaagagca ggctaaatat 960

tatgaattag cacggaaaga aagacagcta catatgcagc tttatccagg ctggtctgca 1020

agagacaatt atggtaagaa aaagaagagg aagagagaga aactacagga atctgcatca 1080

ggtggaaaac gaagctcatt cccaacgtgc aaagccaagg cagcgacccc aggacctctt 1140

ctggagatgg aagcttgttg a 1161

<210> 24
<211> 1122
<212> DNA
<213> Homo sapiens

<220>
<221> GLUL
<222> (1)..(1122)

<400> 24
atgaccacct cagcaagttc ccacttaaat aaaggcatca agcaggtgta catgtccctg 60

cctcagggtg agaaagtcca ggccatgtat atctggatcg atggtactgg agaaggactg 120

cgctgcaaga cccggaccct ggacagtgag cccaagtgtg tggaagagtt gcctgagtgg 180

aatttcgatg gctctagtac tttacagtct gagggttcca acagtgacat gtatctcgtg 240

cctgctgcca tgtttcggga ccccttccgt aaggacccta acaagctggt gttatgtgaa 300

gttttcaagt acaatcgaag gcctgcagag accaatttga ggcacacctg taaacggata 360

atggacatgg tgagcaacca gcacccctgg tttggcatgg agcaggagta taccctcatg 420

gggacagatg ggcacccctt tggttggcct tccaacggct tcccagggcc ccagggtcca 480

tattactgtg gtgtgggagc agacagagcc tatggcaggg acatcgtgga ggcccattac 540

cgggcctgct tgtatgctgg agtcaagatt gcggggacta atgccgaggt catgcctgcc 600

cagtgggaat ttcagattgg accttgtgaa ggaatcagca tgggagatca tctctgggtg 660

gcccgtttca tcttgcatcg tgtgtgtgaa gactttggag tgatagcaac ctttgatcct 720

aagcccattc ctgggaactg gaatggtgca ggctgccata ccaacttcag caccaaggcc 780

atgcgggagg agaatggtct gaagtacatc gaggaggcca ttgagaaact aagcaagcgg 840

caccagtacc acatccgtgc ctatgatccc aagggaggcc tggacaatgc ccgacgtcta 900

actggattcc atgaaacctc caacatcaac gacttttctg ctggtgtagc caatcgtagc 960

gccagcatac gcattccccg gactgttggc caggagaaga agggttactt tgaagatcgt 1020

cgcccctctg ccaactgcga cccctttcgg gtgacagaag ccctcatccg cacgtgtctt 1080

ctcaatgaaa ccggcgatga gcccttccag tacaaaaatt aa 1122

<210> 25
<211> 501
<212> DNA
<213> Homo sapiens

<220>
<221> REG1B
<222> (1)..(501)

<400> 25
atggctcaga ccaactcgtt cttcatgctg atctcctccc tgatgttcct gtctctgagc 60

caaggccagg agtcccagac agagctgcct aatccccgaa tcagctgccc agaaggcacc 120

aatgcctatc gctcctactg ctactacttt aatgaagacc ctgagacctg ggttgatgca 180

gatctctatt gccagaacat gaattcaggc aacctggtgt ctgtgctcac ccaggcggag 240

ggtgccttcg tggcctcact gattaaggag agtagcactg atgacagcaa tgtctggatt 300

ggcctccatg acccaaaaaa gaaccgccgc tggcactgga gtagtgggtc cctggtctcc 360

tacaagtcct gggacactgg atccccgagc agtgctaatg ctggctactg tgcaagcctg 420

acttcatgct caggattcaa gaaatggaag gatgaatctt gtgagaagaa gttctccttt 480

gtttgcaagt tcaaaaacta g 501

<210> 26
<211> 1791
<212> DNA
<213> Homo sapiens

<220>
<221> TCF7L2
<222> (1)..(1791)

<400> 26
atgccgcagc tgaacggcgg tggaggggat gacctaggcg ccaacgacga actgatttcc 60

ttcaaagacg agggcgaaca ggaggagaag agctccgaaa actcctcggc agagagggat 120

ttagctgatg tcaaatcgtc tctagtcaat gaatcagaaa cgaatcaaaa cagctcctcc 180

gattccgagg cggaaagacg gcctccgcct cgctccgaaa gtttccgaga caaatcccgg 240

gaaagtttgg aagaagcggc caagaggcaa gatggagggc tctttaaggg gccaccgtat 300

cccggctacc ccttcatcat gatccccgac ctgacgagcc cctacctccc caacggatcg 360

ctctcgccca ccgcccgaac ctatctccag atgaaatggc cactgcttga tgtccaggca 420

gggagcctcc agagtagaca agccctcaag gatgcccggt ccccatcacc ggcacacatt 480

gtctctaaca aagtgccagt ggtgcagcac cctcaccatg tccaccccct cacgcctctt 540

atcacgtaca gcaatgaaca cttcacgccg ggaaacccac ctccacactt accagccgac 600

gtagacccca aaacaggaat cccacggcct ccgcaccctc cagatatatc cccgtattac 660

ccactatcgc ctggcaccgt aggacaaatc ccccatccgc taggatggtt agtaccacag 720

caaggtcaac cagtgtaccc aatcacgaca ggaggattca gacaccccta ccccacagct 780

ctgaccgtca atgcttccat gtccaggttc cctccccata tggtcccacc acatcatacg 840

ctacacacga cgggcattcc gcatccggcc atagtcacac caacagtcaa acaggaatcg 900

tcccagagtg atgtcggctc actccatagt tcaaagcatc aggactccaa aaaggaagaa 960

gaaaagaaga agccccacat aaagaaacct cttaatgcat tcatgttgta tatgaaggaa 1020

atgagagcaa aggtcgtagc tgagtgcacg ttgaaagaaa gcgcggccat caaccagatc 1080

cttgggcgga ggtggcatgc actgtccaga gaagagcaag cgaaatacta cgagctggcc 1140

cggaaggagc gacagcttca tatgcaactg taccccggct ggtccgcgcg ggataactat 1200

ggaaagaaga agaagaggaa aagggacaag cagccgggag agaccaatga acacagcgaa 1260

tgtttcctaa atccttgcct ttcacttcct ccgattacag acctgagcgc tcctaagaaa 1320

tgccgagcgc gctttggcct tgatcaacag aataactggt gcggcccttg caggagaaaa 1380

aaaaagtgcg ttcgctacat acaaggtgaa ggcagctgcc tcagcccacc ctcttcagat 1440

ggaagcttac tagattcgcc tccccctcc ccgaacctgc taggctcccc tccccgagac 1500

gccaagtcac agactgagca gacccagcct ctgtcgctgt ccctgaagcc cgaccccctg 1560

gcccacctgt ccatgatgcc tccgccaccc gccctcctgc tcgctgaggc cacccacaag 1620

gcctccgccc tctgtcccaa cggggccctg gacctgcccc cagccgcttt gcagcctgcc 1680

gccccctcct catcaattgc acagccgtcg acttcttcct tacattccca cagctccctg 1740

gccgggaccc agccccagcc gctgtcgctc gtcaccaagt ctttagaata g 1791

<210> 27
<211> 4560
<212> DNA
<213> Homo sapiens

<220>
<221> COL18A1
<222> (1)..(4560)

<400> 27
atggctccct acccctgtgg ctgccacatc ctgctgctgc tcttctgctg cctggcggct 60

gcccgggcca acctgctgaa cctgaactgg ctttggttca ataatgagga caccagccat 120

gcagctacca cgatccctga gccccagggg cccctgcctg tgcagcccac agcagatacc 180

accacacacg tgaccccccg gaatggttcc acagagccag cgacagcccc tggcagccct 240

gagccaccct cagagctgct ggaagatggc caggacaccc ccacttctgc cgagagcccg 300

gacgcgccag aggagaacat tgccggtgtc ggagccgaga tcctgaacgt ggccaaaggc 360

atccggagct tcgtccagct gtggaatgac actgtcccca ctgagagctt ggccagggcg 420

gaaaccctgg tcctggagac tcctgtgggc ccccttgccc tcgctgggcc ttccagcacc 480

ccccaggaga atgggaccac tctctggccc agccgtggca ttcctagctc tccgggcgcc 540

cacacaaccg aggctggcac cttgcctgca cccaccccat cgcctccctc cctgggcagg 600

ccctgggcac cactcacggg gccctcagtg ccaccaccat cttcagagcg catcagcgag 660

gaggtggggc tgctgcagct ccttggggac cccccgcccc agcaggtcac ccagacggat 720

gaccccgacg tcgggctggc ctacgtcttt gggccagatg ccaacagtgg ccaagtggcc 780

cggtaccact tccccagcct cttcttccgt gacttctcac tgctgttcca catccggcca 840

gccacagagg gcccaggggt gctgttcgcc atcacggact cggcgcaggc catggtcttg 900

ctgggcgtga agctctctgg ggtgcaggac gggcaccagg acatctccct gctctacaca 960

gaaccaggtg caggccagac ccacacagcc gccagcttcc ggctccccgc cttcgtcggc 1020

cagtggacac acttagccct cagtgtggca ggtggctttg tggccctcta cgtggactgt 1080

gaggagttcc agagaatgcc gcttgctcgg tcctcacggg gcctggagct ggagcctggc 1140

gccgggctct tcgtggctca ggcgggggga gcggaccctg acaagttcca gggggtgatc 1200

gctgagctga aggtgcgcag ggacccccag gtgagcccca tgcactgcct ggacgaggaa 1260

ggcgatgact cagatggggc atccggagac tctggcagcg ggctcgggga cgcccgggag 1320

cttctcaggg aggagacggg cgcggcccta aaacccaggc tccccgcgcc accccccgtc 1380

accacgccac ccttggctgg aggcagcagc acggaagatt ccagaagtga agaagtcgag 1440

gagcagacca cggtggcttc gttaggagct cagacacttc ctggctcaga ttctgtctcc 1500

acgtgggacg ggagtgtccg gacccctggg ggccgcgtga aagagggcgg cctgaagggg 1560

cagaaagggg agccaggtgt tccgggccca cctggccggg caggcccccc aggatcccca 1620

tgcctacctg gtcccccggg tctcccgtgc ccagtgagtc ccctgggtcc tgcaggccca 1680

gcgttgcaaa ctgtccccgg accacaagga cccccagggc ctccggggag ggacggcacc 1740

cctggaaggg acggcgagcc gggcgacccc ggtgaagacg gaaagccggg cgacaccggg 1800

ccacaaggct tccccgggac tccaggggac gtaggtccca agggcgacaa gggagaccct 1860

ggggttggag agagagggcc cccaggaccc caaggcctcc cagggccccc aggaccctcc 1920

ttcagacacg acaagctgac cttcattgac atggagggat ctggcttcgg gggcgatctg 1980

gaggccctgc ggggtcctcg aggcttccct ggacctcccg gacccccggg tgtcccaggc 2040

ctgcccggcg agccaggccg ctttggggtg aacagctccg acgtcccagg acccgccggc 2100

cttcctggtg tgcctgggcg cgagggtccc cccgggtttc ctggcctccc gggaccccca 2160

ggccctccgg gaagagaggg gcccccagga aggactgggc agaaaggcag cctgggtgaa 2220

gcaggcgccc caggacataa ggggagcaag ggagcccccg gtcctgctgg tgctcgtggg 2280

gagagcggcc tggcaggagc ccccggacct gctggaccac caggcccccc tgggccccct 2340

gggcccccag gaccaggact ccccgctgga tttgatgaca tggaaggctc cggggggccc 2400

ttctggtcaa cagcccgaag cgctgatggg ccacagggac ctcccggcct gccgggactt 2460

aagggggatc ctggcgtgcc tgggctgccg ggggcgaagg gagaagttgg agcagatgga 2520

gtccccgggt tccccggcct ccctggcaga gagggcattg ctgggcccca ggggccaaag 2580

ggagacagag gcagccgggg agaaaaggga gatccaggga aggacggagt cgggcagccg 2640

ggcctccctg gcccccccgg acccccggga cctgtggtct acgtgtcgga gcaggacgga 2700

tccgtcctga gcgtgccggg acctgagggc cggccgggtt tcgcaggctt tcccggacct 2760

gcaggaccca agggcaacct gggctctaag ggcgaacgag gctccccggg acccaagggt 2820

gagaagggtg aaccgggcag catcttcagc cccgacggcg gtgccctggg ccctgcccag 2880

aaaggagcca agggagagcc gggcttccga ggacccccgg gtccatacgg acggccgggg 2940

tacaagggag agattggctt tcctggacgg ccgggtcgcc ccgggatgaa cggattgaaa 3000

ggagagaaag gggagccggg agatgccagc cttggatttg gcatgagggg aatgcccggc 3060

cccccaggac ctccagggcc cccaggccct ccagggactc ctgtttacga cagcaatgtg 3120

tttgctgagt ccagccgccc cgggcctcca ggattgccag ggaatcaggg ccctccagga 3180

cccaagggcg ccaaaggaga agtgggcccc cccggaccac cagggcagtt tccgtttgac 3240

tttcttcagt tggaggctga aatgaagggg gagaagggag accgaggtga tgcaggacag 3300

aaaggcgaaa ggggggagcc cgggggcggc ggtttcttcg gctccagcct gcccggcccc 3360

cccggccccc caggcccccc aggcccacgt ggctaccctg ggattccagg tcccaaggga 3420

gagagcatcc ggggccagcc cggcccacct ggacctcagg gaccccccgg catcggctac 3480

gaggggcgcc agggccctcc cggcccccca ggccccccag ggcccccttc atttcctggc 3540

cctcacaggc agactatcag cgttcccggc cctccgggcc cccctgggcc ccctgggccc 3600

cctggaacca tgggcgcctc ctcaggggtg aggctctggg ctacacgcca ggccatgctg 3660

ggccaggtgc acgaggttcc cgagggctgg ctcatcttcg tggccgagca ggaggagctc 3720

tacgtccgcg tgcagaacgg gttccggaag gtccagctgg aggcccggac accactccca 3780

cgagggacgg acaatgaagt ggccgccttg cagccccccg tggtgcagct gcacgacagc 3840

aacccctacc cgcggcggga gcacccccac cccaccgcgc ggccctggcg ggcagatgac 3900

atcctggcca gcccccctcg cctgcccgag ccccagccct accccggagc cccgcaccac 3960

agctcctacg tgcacctgcg gccggcgcga cccacaagcc cacccgccca cagccaccgc 4020

gacttccagc cggtgctcca cctggttgcg ctcaacagcc ccctgtcagg cggcatgcgg 4080

ggcatccgcg gggccgactt ccagtgcttc cagcaggcgc gggccgtggg gctggcgggc 4140

accttccgcg ccttcctgtc ctcgcgcctg caggacctgt acagcatcgt gcgccgtgcc 4200

gaccgcgcag ccgtgcccat cgtcaacctc aaggacgagc tgctgtttcc cagctgggag 4260

gctctgttct caggctctga gggtccgctg aagcccgggg cacgcatctt ctcctttgac 4320

ggcaaggacg tcctgaggca ccccacctgg ccccagaaga gcgtgtggca tggctcggac 4380

cccaacgggc gcaggctgac cgagagctac tgtgagacgt ggcggacgga ggctccctcg 4440

gccacgggcc aggcctcctc gctgctgggg ggcaggctcc tggggcagag tgccgcgagc 4500

tgccatcacg cctacatcgt gctctgcatt gagaacagct tcatgactgc ctccaagtag 4560

<210> 28
<211> 1296
<212> DNA
<213> Homo sapiens

<220>
<221> BMP7
<222> (1)..(1296)

<400> 28
atgcacgtgc gctcactgcg agctgcggcg ccgcacagct tcgtggcgct ctgggcaccc 60

ctgttcctgc tgcgctccgc cctggccgac ttcagcctgg acaacgaggt gcactcgagc 120

ttcatccacc ggcgcctccg cagccaggag cggcgggaga tgcagcgcga gatcctctcc 180

attttgggct tgccccaccg cccgcgcccg cacctccagg gcaagcacaa ctcggcaccc 240

atgttcatgc tggacctgta caacgccatg gcggtggagg agggcggcgg gcccggcggc 300

cagggcttct cctaccccta caaggccgtc ttcagtaccc agggcccccc tctggccagc 360

ctgcaagata gccatttcct caccgacgcc gacatggtca tgagcttcgt caacctcgtg 420

gaacatgaca aggaattctt ccacccacgc taccaccatc gagagttccg gtttgatctt 480

tccaagatcc cagaagggga agctgtcacg gcagccgaat tccggatcta caaggactac 540

atccgggaac gcttcgacaa tgagacgttc cggatcagcg tttatcaggt gctccaggag 600

cacttgggca gggaatcgga tctcttcctg ctcgacagcc gtaccctctg ggcctcggag 660

gagggctggc tggtgtttga catcacagcc accagcaacc actgggtggt caatccgcgg 720

cacaacctgg gcctgcagct ctcggtggag acgctggatg ggcagagcat caaccccaag 780

ttggcgggcc tgattgggcg gcacgggccc cagaacaagc agcccttcat ggtggctttc 840

ttcaaggcca cggaggtcca cttccgcagc atccggtcca cggggagcaa acagcgcagc 900

cagaaccgct ccaagacgcc caagaaccag gaagccctgc ggatggccaa cgtggcagag 960

aacagcagca gcgaccagag gcaggcctgt aagaagcacg agctgtatgt cagcttccga 1020

gacctgggct ggcaggactg gatcatcgcg cctgaaggct acgccgccta ctactgtgag 1080

ggggagtgtg ccttccctct gaactcctac atgaacgcca ccaaccacgc catcgtgcag 1140

acgctggtcc acttcatcaa cccggaaacg gtgcccaagc cctgctgtgc gcccacgcag 1200

ctcaatgcca tctccgtcct ctacttcgat gacagctcca acgtcatcct gaagaaatac 1260

agaaacatgg tggtccgggc ctgtggctgc cactag 1296

<210> 29

<211>  1725
<212>  DNA
<213>  Homo sapiens

<220>
<221>  SLC1A2
<222>  (1)..(1725)

<400>  29
atggcatcta cggaaggtgc caacaatatg cccaagcagg tggaagtgcg aatgcacgac      60

agtcatcttg gctcagagga acccaagcac cggcacctgg gcctgcgcct gtgtgacaag     120

ctggggaaga atctgctgct caccctgacg gtgtttggtg tcatcctggg agcagtgtgt     180

ggagggcttc ttcgcttggc atctcccatc caccctgatg tggttatgtt aatagccttc     240

ccaggggata tactcatgag gatgctaaaa atgctcattc tccctctaat catctccagc     300

ttaatcacag ggttgtcagg cctggatgct aaggctagtg gccgcttggg cacgagagcc     360

atggtgtatt acatgtccac gaccatcatt gctgcagtac tgggggtcat tctggtcttg     420

gctatccatc caggcaatcc caagctcaag aagcagctgg ggcctgggaa gaagaatgat     480

gaagtgtcca gcctggatgc cttcctggac cttattcgaa atctcttccc tgaaaacctt     540

gtccaagcct gctttcaaca gattcaaaca gtgacgaaga aagtcctggt tgcaccaccg     600

ccggacgagg aggccaacgc aaccagcgct gttgtctctc tgttgaacga gactgtgact     660

gaggtgccgg aggagactaa gatggttatc aagaagggcc tggagttcaa ggatgggatg     720

aacgtcttag gtctgatagg gtttttcatt gcttttggca tcgctatggg gaagatggga     780

gatcaggcca agctgatggt ggatttcttc aacattttga atgagattgt aatgaagtta     840

gtgatcatga tcatgtggta ctctcccctg ggtatcgcct gcctgatctg tggaaagatc     900

attgcaatca aggacttaga agtggttgct aggcaactgg ggatgtacat ggtaacagtg     960

atcataggcc tcatcatcca cgggggcatc tttctcccct tgatttactt tgtagtgacc    1020

aggaaaaacc ccttctcctt ttttgctggc attttccaag cttggatcac tgccctgggc    1080

accgcttcca gtgctggaac tttgcctgtc acctttcgtt gcctggaaga aaatctgggg    1140

attgataagc gtgtgactag attcgtcctt cctgttggag caaccattaa catggatggt    1200

acagcccttt atgaagcggt agccgccatc tttatagccc aaatgaatgg tgttgtcctg    1260

gatggaggac agattgtgac tgtaagcctc acagccaccc tggcaagcgt cggcgcggcc    1320

agtatcccca gtgccgggct ggtcaccatg ctcctcattc tgacagccgt gggcctgcca    1380

acagaggaca tcagcctgct ggtggctgtg gactggctgc tggacaggat gagaacttca    1440

gtcaatgttg tgggtgactc ttttggggct gggatagtct atcacctctc caagtctgag    1500

ctggatacca ttgactccca gcatcgagtg catgaagata ttgaaatgac caagactcaa    1560

tccatttatg atgacatgaa gaaccacagg gaaagcaact ctaatcaatg tgtctatgct    1620

gcacacaact ctgtcatagt agatgaatgc aaggtaactc tggcagccaa tggaaagtca    1680

gccgactgca gtgttgagga agaaccttgg aaacgtgaga aataa 1725

<210> 30
<211> 1389
<212> DNA
<213> Homo sapiens

<220>
<221> ADRA2C
<222> (1)..(1389)

<400> 30
atggcgtccc cggcgctggc ggcggcgctg gcggtggcgg cagcggcggg ccccaatgcg 60

agcggcgcgg gcgagagggg cagcggcggg gttgccaatg cctcgggggc ttcctggggg 120

ccgccgcgcg gccagtactc ggcgggcgcg gtggcagggc tggctgccgt ggtgggcttc 180

ctcatcgtct tcaccgtggt gggcaacgtg ctggtggtga tcgccgtgct gaccagccgg 240

gcgctgcgcg cgccacagaa cctcttcctg gtgtcgctgg cctcggccga catcctggtg 300

gccacgctgg tcatgccctt ctcgttggcc aacgagctca tggcctactg gtacttcggg 360

caggtgtggt gcggcgtgta cctggcgctc gatgtgctgt tttgcacctc gtcgatcgtg 420

catctgtgtg ccatcagcct ggaccgctac tggtcggtga cgcaggccgt cgagtacaac 480

ctgaagcgca caccacgccg cgtcaaggcc accatcgtgg ccgtgtggct catctcggcc 540

gtcatctcct tcccgccgct ggtctcgctc taccgccagc ccgacggcgc cgcctacccg 600

cagtgcggcc tcaacgacga gacctggtac atcctgtcct cctgcatcgg ctccttcttc 660

gcgccctgcc tcatcatggg cctggtctac gcgcgcatct accgagtggc caagctgcgc 720

acgcgcacgc tcagcgagaa gcgcgccccc gtgggccccg acggtgcgtc cccgactacc 780

gaaaacgggc tgggcgcggc ggcaggcgca ggcgagaacg ggcactgcgc gcccccgccc 840

gccgacgtgg agccggacga gagcagcgca gcggccgaga ggcggcggcg ccggggcgcg 900

ttgcggcggg gcgggcggcg gcgagcgggc gcggaggggg gcgcgggcgg tgcggacggg 960

cagggggcgg ggccgggggc ggctgagtcg ggggcgctga ccgcctccag gtccccgggg 1020

cccggtggcc gcctgtcgcg cgccagctcg cgctccgtcg agttcttcct gtcgcgccgg 1080

cgccgggcgc gcagcagcgt gtgccgccgc aaggtggccc aggcgcgcga gaagcgcttc 1140

acctttgtgc tggctgtggt catgggcgtg ttcgtgctct gctggttccc cttcttcttc 1200

agctacagcc tgtacggcat ctgccgcgag gcctgccagg tgcccggccc gctcttcaag 1260

ttcttcttct ggatcggcta ctgcaacagc tcgctcaacc cggtcatcta cacggtcttc 1320

aaccaggatt tccggcgatc ctttaagcac atcctcttcc gacggaggag aaggggcttc 1380

aggcagtga 1389

<210> 31

<211>  1434
<212>  DNA
<213>  Homo sapiens

<220>
<221>  PPARG
<222>  (1)..(1434)

<400>  31
atgaccatgg ttgacacaga gatgccattc tggcccacca actttgggat cagctccgtg      60

gatctctccg taatggaaga ccactcccac tcctttgata tcaagccctt cactactgtt     120

gacttctcca gcatttctac tccacattac gaagacattc cattcacaag aacagatcca     180

gtggttgcag attacaagta tgacctgaaa cttcaagagt accaaagtgc aatcaaagtg     240

gagcctgcat ctccacctta ttattctgag aagactcagc tctacaataa gcctcatgaa     300

gagccttcca actccctcat ggcaattgaa tgtcgtgtct gtggagataa agcttctgga     360

tttcactatg gagttcatgc ttgtgaagga tgcaagggtt tcttccggag aacaatcaga     420

ttgaagctta tctatgacag atgtgatctt aactgtcgga tccacaaaaa aagtagaaat     480

aaatgtcagt actgtcggtt tcagaaatgc cttgcagtgg ggatgtctca taatgccatc     540

aggtttgggc ggatgccaca ggccgagaag gagaagctgt tggcggagat ctccagtgat     600

atcgaccagc tgaatccaga gtccgctgac ctccgggccc tggcaaaaca tttgtatgac     660

tcatacataa agtccttccc gctgaccaaa gcaaaggcga gggcgatctt gacaggaaag     720

acaacagaca aatcaccatt cgttatctat gacatgaatt ccttaatgat gggagaagat     780

aaaatcaagt tcaaacacat cacccccctg caggagcaga gcaaagaggt ggccatccgc     840

atctttcagg gctgccagtt tcgctccgtg gaggctgtgc aggagatcac agagtatgcc     900

aaaagcattc ctggttttgt aaatcttgac ttgaacgacc aagtaactct cctcaaatat     960

ggagtccacg agatcattta cacaatgctg gcctccttga tgaataaaga tggggttctc    1020

atatccgagg gccaaggctt catgacaagg gagtttctaa agagcctgcg aaagcctttt    1080

ggtgacttta tggagcccaa gtttgagttt gctgtgaagt tcaatgcact ggaattagat    1140

gacagcgact tggcaatatt tattgctgtc attattctca gtggagaccg cccaggtttg    1200

ctgaatgtga agcccattga agacattcaa gacaacctgc tacaagccct ggagctccag    1260

ctgaagctga accaccctga gtcctcacag ctgtttgcca agctgctcca gaaaatgaca    1320

gacctcagac agattgtcac ggaacacgtg cagctactgc aggtgatcaa gaagacggag    1380

acagacatga gtcttcaccc gctcctgcag gagatctaca aggacttgta ctag          1434

<210>  32
<211>  801
<212>  DNA
<213>  Homo sapiens

<220>
<221>  DKK1
<222>  (1)..(801)

<400>  32
atgatggctc tgggcgcagc gggagctacc cgggtctttg tcgcgatggt agcggcggct      60

ctcggcggcc accctctgct gggagtgagc gccaccttga actcggttct caattccaac     120

gctatcaaga acctgccccc accgctgggc ggcgctgcgg ggcacccagg ctctgcagtc     180

agcgccgcgc cgggaatcct gtacccgggc gggaataagt accagaccat tgacaactac     240

cagccgtacc cgtgcgcaga ggacgaggag tgcggcactg atgagtactg cgctagtccc     300

acccgcggag gggacgcagg cgtgcaaatc tgtctcgcct gcaggaagcg ccgaaaacgc     360

tgcatgcgtc acgctatgtg ctgccccggg aattactgca aaaatggaat atgtgtgtct     420

tctgatcaaa atcatttccg aggagaaatt gaggaaacca tcactgaaag ctttggtaat     480

gatcatagca ccttggatgg gtattccaga agaaccacct tgtcttcaaa aatgtatcac     540

accaaaggac aagaaggttc tgtttgtctc cggtcatcag actgtgcctc aggattgtgt     600

tgtgctagac acttctggtc caagatctgt aaacctgtcc tgaaagaagg tcaagtgtgt     660

accaagcata ggagaaaagg ctctcatgga ctagaaatat tccagcgttg ttactgtgga     720

gaaggtctgt cttgccggat acagaaagat caccatcaag ccagtaattc ttctaggctt     780

cacacttgtc agagacacta a                                               801


<210>  33
<211>  1896
<212>  DNA
<213>  Homo sapiens


<220>
<221>  HNF1A
<222>  (1)..(1896)

<400>  33
atggtttcta aactgagcca gctgcagacg gagctcctgg cggccctgct cgagtcaggg      60

ctgagcaaag aggcactgat ccaggcactg ggtgagccgg ggccctacct cctggctgga     120

gaaggccccc tggacaaggg ggagtcctgc ggcggcggtc gaggggagct ggctgagctg     180

cccaatgggc tgggggagac tcggggctcc gaggacgaga cggacgacga tggggaagac     240

ttcacgccac ccatcctcaa agagctggag aacctcagcc ctgaggaggc ggcccaccag     300

aaagccgtgg tggagaccct tctgcaggag gacccgtggc gtgtggcgaa gatggtcaag     360

tcctacctgc agcagcacaa catcccacag cgggaggtgg tcgataccac tggcctcaac     420

cagtcccacc tgtcccaaca cctcaacaag ggcactccca tgaagacgca gaagcgggcc     480

gccctgtaca cctggtacgt ccgcaagcag cgagaggtgg cgcagcagtt cacccatgca     540

gggcagggag ggctgattga agagcccaca ggtgatgagc taccaaccaa gaaggggcgg     600

aggaaccgtt tcaagtgggg cccagcatcc cagcagatcc tgttccaggc ctatgagagg 660

cagaagaacc ctagcaagga ggagcgagag acgctagtgg aggagtgcaa tagggcggaa 720

tgcatccaga gaggggtgtc cccatcacag gcacaggggc tgggctccaa cctcgtcacg 780

gaggtgcgtg tctacaactg gtttgccaac cggcgcaaag aagaagcctt ccggcacaag 840

ctggccatgg acacgtacag cgggcccccc ccagggccag gcccgggacc tgcgctgccc 900

gctcacagct cccctggcct gcctccacct gccctctccc ccagtaaggt ccacggtgtg 960

cgctatggac agcctgcgac cagtgagact gcagaagtac cctcaagcag cggcggtccc 1020

ttagtgacag tgtctacacc cctccaccaa gtgtccccca cgggcctgga gcccagccac 1080

agcctgctga gtacagaagc caagctggtc tcagcagctg gggcccccct cccccctgtc 1140

agcaccctga cagcactgca cagcttggag cagacatccc caggcctcaa ccagcagccc 1200

cagaacctca tcatggcctc acttcctggg gtcatgacca tcgggcctgg tgagcctgcc 1260

tccctgggtc ctacgttcac caacacaggt gcctccaccc tggtcatcgg cctggcctcc 1320

acgcaggcac agagtgtgcc ggtcatcaac agcatgggca gcagcctgac caccctgcag 1380

cccgtccagt tctcccagcc gctgcacccc tcctaccagc agccgctcat gccacctgtg 1440

cagagccatg tgacccagag ccccttcatg gccaccatgg ctcagctgca gagcccccac 1500

gccctctaca gccacaagcc cgaggtggcc cagtacaccc acacgggcct gctcccgcag 1560

actatgctca tcaccgacac caccaacctg agcgccctgg ccagcctcac gcccaccaag 1620

caggtcttca cctcagacac tgaggcctcc agtgagtccg ggcttcacac gccggcatct 1680

caggccacca ccctccacgt ccccagccag gaccctgcca gcatccagca cctgcagccg 1740

gcccaccggc tcagcgccag ccccacagtg tcctccagca gcctggtgct gtaccagagc 1800

tcagactcca gcaatggcca gagccacctg ctgccatcca accacagcgt catcgagacc 1860

ttcatctcca cccagatggc ctcttcctcc cagtaa 1896

<210> 34
<211> 456
<212> DNA
<213> Homo sapiens

<220>
<221> LECT2
<222> (1)..(456)

<400> 34
atgttttcca ccaaagccct ccttttggct ggtctgattt ctaccgcact ggcagggcca 60

tgggctaata tatgtgctgg caagtcttcc aatgagatcc ggacgtgtga ccgccatggc 120

tgtggacagt actctgctca aagaagtcag aggcctcacc agggtgtgga catcttgtgc 180

tctgctggat ctactgtgta cgcaccattc actggaatga ttgtgggcca ggagaaacct 240

tatcaaaaca agaatgctat caataatggt gttcgaatat ctggaagagg tttttgtgtc 300

aaaatgttct acattaagcc aattaagtat aaaggtccta ttaagaaggg agaaaaactt 360

ggaactctat tgcccttgca gaaagtttat cctggcatac aatcgcatgt gcacattgaa 420

aactgtgact cgagtgaccc tactgcatac ctgtaa 456